# EUROPÄISCHE PATENTANMELDUNG

(11) **EP 3 498 721 A1**
(43) Veröffentlichungstag der Anmeldung: **19.06.2019**
(21) Anmeldenummer: 17207806.5
(22) Anmeldetag: 15.12.2017
(51) Int. Cl.: C07F 17/02, C07C 243/42, C07F 5/00, C07F 5/06, C23C 16/00, C30B 25/00

(54) **METALLKOMPLEXE MIT TRIAZENIDOLIGANDEN UND DEREN VERWENDUNGEN ZUR ABSCHEIDUNG VON METALLEN AUS DER GASPHASE**

(71) Anmelder: UMICORE AG & CO. KG, 63457 Hanau-Wolfgang (DE)
(72) Erfinder: Die Erfindernennung liegt noch nicht vor

(57) **Zusammenfassung**

Die Erfindung betrifft die Verwendung eines Metallkomplexes, der mindestens einen Liganden der Formel R¹-N₃-R² aufweist, wobei R¹ und R² Kohlenwasserstoffreste sind, zum Abscheiden des Metalls oder einer Verbindung des Metalls aus der Gasphase. Die Erfindung betrifft außerdem Verfahren zur Abscheidung von Metallen aus den Metallkomplexen, sowie Metallkomplexe, substituierte Triazenverbindungen und Verfahren zu deren Herstellung.

## Beschreibung

Die Erfindung betrifft die Verwendung eines Metallkomplexes, der mindestens einen Liganden der Formel R¹-N₃-R² aufweist, wobei R¹ und R² Kohlenwasserstoffreste, insbesondere Alkylreste, sind, zum Abscheiden des Metalls oder einer Verbindung des Metalls aus der Gasphase. Die Erfindung betrifft außerdem Verfahren zur Abscheidung von Metallen aus den Metallkomplexen, sowie Metallkomplexe, substituierte Triazenido-Verbindungen und Verfahren zu deren Herstellung.

### Stand der Technik

Die metallorganische Gasphasenabscheidung, und insbesondere die metallorganische Gasphasenepitaxie sind wichtige Verfahren, um dünne Schichten von Metallen oder Metallverbindungen auf Substraten zu erzeugen. Diese Verfahren werden insbesondere in der Halbleiterindustrie eingesetzt. Dabei werden metallorganische Verbindungen, gegebenenfalls in Verbindung mit weiteren reaktiven Verbindungen, in Prozesskammern eingeführt, wo bei Unterdruck bis Normaldruck eine Reaktion auf der Oberfläche erwärmter Substrate stattfindet, die zur Abscheidung der Schicht führt. Mit solchen Verfahren kann eine Vielzahl von metallhaltigen Schichten, wie Halbleiterkristalle, amorphen Schichten, metallische Verbindungen oder Metallschichten, auf Substraten abgeschieden werden. Für eine Übersicht wird beispielhaft auf das "Handbook of Thin Film Deposition - Processes and Technologies", 2. Auflage 2001, Herausgeber: Krishna Seshan, Kapitel 4, S. 151-203, von J. Zilko verwiesen.

Für viele Anwendungen, insbesondere in dem Halbleiterbereich, ist es unabdingbar, dass solche metallhaltigen Schichten in hoher Reinheit erzeugt werden. Bereits geringfügige Spuren von Verunreinigungen können den bestimmungsgemäßen Gebrauch beeinträchtigen.

Zur Erzeugung hochreiner metallhaltiger Schichten sind jedoch nur sehr wenige metallorganische Vorläuferverbindungen geeignet. Dies liegt oft daran, dass eine geeignete metallorganische Vorläuferverbindung bei hoher Temperatur in die Gasphase überführbar sein sollte. Viele metallorganische Verbindungen sind jedoch thermisch instabil. Sie zersetzen sich (zerfallen) bei höheren Temperaturen und können daher nicht durch Erwärmen oder auf andere Weise in die Gasphase überführt werden. Problematisch ist dabei, dass organometallische Verbindungen oft Feststoffe sind, die relativ hohe Verdampfungstemperaturen aufweisen, bei denen bereits Zersetzungsprozesse, unter anderem wegen den reaktiven organischen Liganden, stattfinden.

Wenn eine metallorganische Verbindung verdampft werden kann, wird üblicherweise die Temperatur in der Gasphase über dem Substrat, also dem Target der Abscheidung, weiter erhöht, bis eine Zersetzung stattfindet. Jedoch sind selbst organometallische Verbindungen, die in die Gasphase überführbar sind, oft nicht zur Abscheidung von Metallen geeignet. Der Grund dafür ist, dass die Abscheidung des Metalls auf der Substratoberfläche derart erfolgen muss, dass keine noch so geringen Verunreinigungen durch Komponenten der Komplexliganden, wie Kohlenstoff, Stickstoff oder Sauerstoff, erfolgt. Auch diese Voraussetzung wird oft nicht erfüllt, weil bei den erforderlichen hohen Zerfallstemperaturen ein Gemisch von hochreaktiven Intermediaten, ionischen Resten und Radikalen entsteht, so dass auf der Substratoberfläche oft unerwünschte Nebenreaktionen stattfinden.

Zur Abscheidung von metallhaltigen Schichten in der Gasphase werden oft Metallalkylverbindungen als Vorläuferverbindungen eingesetzt. Eine Übersicht über geeignete Verbindungen ist in der oben zitierten Publikation von J. Zilko enthalten. Allerdings weisen die Alkylverbindungen vieler Metalle oft Eigenschaften auf, welche die Eignung für Abscheidungsreaktionen in der Gasphase beeinträchtigen. Dabei ist zum einen nachteilig, dass viele reine Metallalkylverbindungen der Übergangsmetalle instabil sind, zum Zerfall neigen und nicht lagerfähig sind. Zum anderen neigen sie dazu, dass anionischer Kohlenstoff, der am Übergangsmetall gebunden ist, in unerwünschter Weise mit in die Schicht eingebaut wird. Sie weisen oft auch relativ hohe Verdampfungstemperaturen auf, was für Anwendungen bei der Gasphasenabscheidung nachteilig ist.

Es besteht daher ein kontinuierlicher Bedarf nach verbesserten Verfahren und Materialien zur Abscheidung von Metallen und metallhaltiger Schichten durch metallorganische Gasphasenabscheidung.

Im Stand der Technik sind metallorganische Komplexe bekannt, die N-Aryl- und N,N'-Diaryl-Triazenidoliganden aufweisen. Allerdings wird im Stand der Technik nicht offenbart, solche Komplexe zur Abscheidung von Metallen aus der Gasphase zu nutzen. Dies ist nicht überraschend, weil solche Metallkomplexe mit diesen Liganden nicht eine ausreichende Flüchtigkeit und Zersetzbarkeit besitzen, die für die Verwendung bei der Abscheidung von Metallen aus der Gasphase zwingend erforderlich ist. Daher würden bei solchen Reaktionen nichtflüchtige Kohlenstoff-Verunreinigungen erhalten.

Metallorganische Komplexe mit Triazenidoliganden, die mit aromatischen Resten substituiert sind, werden in einer Vielzahl von Publikationen beschrieben. Allerdings betreffen nahezu alle diese Publikationen lediglich die Synthese und Struktur solcher Komplexe, während praktische Anwendungen der Verbindungen nur in Ausnahmefällen beschrieben werden.

Dabei sind Methoden bekannt, um verschieden substituierte Vorläuferverbindungen für Triazenid-Anionen bereitzustellen. Im Jahr 1981 beschrieben Brand und Roberts die elektronische Konfiguration von 1,3-Dialkyltriazenyl-Radikalen und erwähnten in einer Fußnote, dass sich Di-*tert*-Butyltriazen ausgehend von *tert*-Butylazid und *tert*-Butyllithium darstellen lässt. Weder experimentelle noch spektroskopische Daten wurden veröffentlicht.^{[1]} Ethyl- und methyl-substituierte Triazene wurden zudem 1983 von Smith und Michejda beschrieben, wobei die Synthesen entweder mit Methyllithium oder dem Grignard-Reagenz Ethylmagnesiumbromid durchgeführt wurden.^{[2]} Darüber hinaus wurden Synthesen unterschiedlicher Alkyl- und Aryl-substituierten Triazene und Untersuchungen zu tautomeren Gleichgewichten in verschiedenen Veröffentlichungen diskutiert.^{[3,4]}

Erdalkali- und Alkalimetall-Verbindungen der Triazene sind ausschließlich von Aryl-substituierten Systemen bekannt. Bei kristallographisch charakterisierten Verbindungen handelt es sich in allen Publikationen um solvatisierte Verbindungen bzw. Verbindungen, in welchen das entsprechende Metall mithilfe von TMEDA oder [15]-Krone-5 stabilisiert wird.^{[6-10]}

Triazenido-Verbindungen mit Elementen der dritten Hauptgruppe sind seit mehreren Jahrzenten literaturbekannt.

Das US-Patent 3,386,985 beschreibt verschiedene metallorganische Verbindungen mit, die verschiedene Triazenidoliganden, unter anderem 1,3-Dimethyltriazen, aufweisen. Es wird vorgeschlagen, die Verbindungen als Kettentransfer-Reagentien oder Inhibitoren in Polymerisationsverfahren einzusetzen. Verfahren zur Abscheidung von Metallen werden nicht beschrieben.

Brinckman et al.^{[11]} beschreiben metallorganische Komplexe mit 1,3-Dimethyltriazenid als Liganden, wobei die Ergebnisse weitgehend mit den in US3,386,985 enthaltenen übereinstimmen. Zu praktischen Verwendungen der Komplexe werden keine Ausführungen gemacht. Die Ausbeuten bei den Herstellungsverfahren liegen bei maximal 76% und sind daher noch verbesserungsbedürftig.

Des Weiteren wurden in der Literatur verschiedene Komplexe, sowohl homoleptische, als auch heteroleptische, mit einem bzw. zwei Aryl-Triazenido-Liganden beschrieben.^{[12-16]}

Im Bereich der Übergangsmetalle ist eine Vielzahl an Triazenido-Verbindungen mit aromatisch substituierten Liganden bekannt, wobei als Zentralatom frühe Übergangsmetalle wie Titan und Zirkonium als auch späte Übergangsmetalle wie Silber und Kupfer untersucht wurden.^{[6,17-23]} Sowohl homoleptische als auch heteroleptische, molekulare oder kationische Cobalt-Komplexe mit aromatisch substituierten Triazenido-Liganden wurden in Zusammenhang mit ESR-Messungen beschrieben. Ebenso wurde von Ruthenium-Verbindungen, in Zusammenhang mit theoretischen Berechnungen, berichtet.^{[24-30]}

Soussi et al.^{[5]} beschreiben die Herstellung metallorganischer Eisenkomplexe, die als Liganden Alkyltriazenide, sowie zur Stabilisierung TMEDA-Liganden (Tetramethylethylendiamin) aufweisen. Die Herstellung der Komplexe erfolgt in einem relativ aufwändigen Verfahren ausgehend von Fe[N(SiMe₃)₂]₂ über die Route des Ligandenaustausches. Die Metallkomplexe werden zur Herstellung von intermetallischen Nanopartikeln verwendet. Das Verdampfungs- und Zersetzungsverhalten der Eisenkomplexe bei Erhöhung der Temperatur wird durch thermogravimetrische Analyse untersucht. Dabei wurde gefunden, dass die vier Metallkomplexe eine geringe thermische Stabilität aufweisen, so dass die Komplexe nicht verdampft werden können. Bei Erhöhung der Temperatur auf mehr als 200°C verbleiben feste Rückstände, die etwa 20 bis 40 Gew.% der Einwaage ausmachen. Zudem zeigt die Gewichtskurve bei Erhöhung der Temperatur auf bis zu 200°C einen unregelmäßigen Verlauf, was typisch für chemische Zerfallsreaktionen ist. Daher sind die Metallkomplexe nicht als Vorläuferverbindungen zur Herstellung von metallhaltigen Beschichtungen aus der Gasphase geeignet.

Verschiedene Seltenerdmetall-Komplexe sind zwar literaturbekannt, jedoch lediglich mit sterisch sehr anspruchsvollen Aryl-Triazenido-Liganden.^{[31-33]}

Es besteht im Stand der Technik ein kontinuierlicher Bedarf nach Verfahren und Verbindungen, die zur Abscheidung von Metallen und metallhaltigen Schichten in der Gasphase verwendbar sind, welche die oben beschriebenen Nachteile überwinden.

### Aufgabe der Erfindung

Der Erfindung liegt die Aufgabe zugrunde, Verfahren und Verbindungen bereitzustellen, welche die oben beschriebenen Nachteile überwinden. Der Erfindung liegt insbesondere die Aufgabe zugrunde, neue und verbesserte Verbindungen bereitzustellen, um Metalle und metallhaltige Schichten aus der Gasphase abzuscheiden.

Die Verbindungen sollen eine relativ hohe Stabilität aufweisen. Dabei sollen die Verbindungen einen hohen Dampfdruck und gleichzeitig einen niedrigen Zersetzungspunkt aufweisen. Dabei soll es möglich sein, die Verbindungen in die Gasphase zu überführen, ohne dass eine wesentliche Zersetzung stattfindet. Nach Überführung in die Gasphase, also bei Temperaturen oberhalb der Sublimationstemperatur oder Verdampfungstemperatur, sollen sich die Verbindungen zersetzen. Bevorzugt soll die Zersetzung dabei bei einer Temperatur erfolgen, die nur wenig oberhalb der Sublimationstemperatur oder Verdampfungstemperatur liegt.

Die Verbindungen sollen allgemein bei Temperaturen, bei denen Abscheidungsverfahren aus der Gasphase üblicherweise durchgeführt werden, und dabei insbesondere von 100°C bis 300°C, eine hohe Stabilität und Volatilität aufweisen. Insbesondere sollen die Verbindungen sublimierbar sein.

Der Erfindung liegt insbesondere die Aufgabe zugrunde, Verbindungen bereitzustellen, die bei Temperaturen bis zu 100°C stabil in die Gasphase überführt werden können, und die bei höheren Temperaturen, beispielsweise im Bereich von 100°C bis 400°C, zerfallen.

Die Verbindungen sollen es ermöglichen, verschiedene Metalle und metallhaltige Verbindungen aus der Gasphase abzuscheiden. Die Beschichtungen sollen hochrein sein. Insbesondere soll bei üblichen Abscheidungsverfahren kein unerwünschter Einbau von Kohlenstoff, Sauerstoff oder Stickstoff in die Beschichtungen stattfinden.

Der Erfindung liegt außerdem die Aufgabe zugrunde, möglichst einfache und effiziente Verfahren zur Herstellung solcher Verbindungen bereitzustellen. Die eingesetzten Reagenzien sollen möglichst leicht zugänglich und sicher handhabbar sein. Die Verfahren sollen mit hohen Ausbeuten durchführbar sein. Die Verfahren sollen in möglichst wenig Stufen zu den gewünschten Produkten führen und sollen unter möglichst sanften Reaktionsbedingungen durchführbar sein. Es sollen auch Verbindungen bereitgestellt werden, aus denen sich die flüchtigen Vorläuferverbindungen auf möglichst einfache Weise herstellen lassen.

### Offenbarung der Erfindung

Überraschenderweise wird die der Erfindung zugrunde liegende Aufgabe gelöst durch Verwendungen, Verfahren, Metallkomplexe und weitere Verbindungen gemäß den Patentansprüchen.

Gegenstand der Erfindung ist die Verwendung eines Metallkomplexes, der mindestens einen Liganden **L** der Formel R¹-N₃-R² aufweist, wobei R¹ und R² Kohlenwasserstoffreste sind, zum Abscheiden des Metalls oder einer Verbindung des Metalls aus der Gasphase. Der Ligand L ist dabei formal ein anionischer Ligand, weil die N₃-Untereinheit eine negative Ladung trägt.

Dabei wird aus der Gasphase eine feste Schicht auf einem Substrat abgeschieden, die aus dem Metall besteht oder dieses enthält. Der Metallkomplex wird dabei als metallorganische Vorläuferverbindung (Precursor) eingesetzt. Das Metall aus dem Metallkomplex wird in elementarer Form oder gezielt in Form einer Verbindung des Metalls abgeschieden. So kann beispielsweise Stickstoff aus dem Liganden oder aus einem geeigneten Reaktionspartner in eine Schicht eingebaut werden, so dass ein Nitrid des abgeschiedenen Metalls entsteht. Bevorzugt wird der Metallkomplex dabei zunächst in die Gasphase überführt, oder es werden zumindest flüchtige Zwischenprodukte in die Gasphase überführt, die das Metall enthalten. In der Gasphase wird die Temperatur bei reduziertem Druck üblicherweise weiter erhöht, so dass eine (weitere) Zersetzung des Metallkomplexes stattfindet. Der Zerfall kann auch auf andere Weise angeregt oder unterstützt werden, beispielsweise durch Strahlung. Die entscheidende Zersetzung des Metallkomplexes sollte erst nach der Überführung in die Gasphase stattfinden. Auf diese Weise wird gewährleistet, dass der Metallkomplex möglichst quantitativ in der Gasphase vorliegt und zur Abscheidung des Metalls oder einer Verbindung des Metalls genutzt werden kann. Außerdem kann regelmäßig die Zahl der unerwünschten Nebenreaktionen verringert werden, wenn ein kontrollierter Zerfall erst in der Gasphase erfolgt.

In einer bevorzugten Ausführungsform weist der Metallkomplex mindestens einen Liganden **L** der Formel R¹-N₃-R²p und mindestens einen weiteren Liganden X auf. Der weitere Ligand X ist bevorzugt ausgewählt aus Halogen, H, CO und Kohlenwasserstoffliganden. Die Kohlenwasserstoffliganden sind insbesondere Alkyl oder Alkenyl mit 1 bis 12 C-Atomen und aromatische Kohlenwasserstoffe mit 5 bis 30 C-Atomen. Der Kohlenwasserstoffligand kann dabei neutral sein oder eine negative Ladung aufweisen. Das Halogen kann F, Cl, Br oder I sein, besonders bevorzugt ist Cl. Wenn X = H ist, liegt eine Hydridokomplex vor. Dieser Komplex kann einen oder mehrere Liganden X aufweisen, beispielsweise einen, zwei oder drei Liganden X. Dabei ist es bevorzugt, dass genau ein oder zwei Liganden X vorhanden sind. Wenn mehr als ein Ligand X vorhanden ist, so können die Liganden X zueinander identisch oder verschieden voneinander sein.

In einer bevorzugten Ausführungsform weist der Metallkomplex mindestens einen Liganden **L** der Formel R¹-N₃-R² auf, bei dem R¹ und R² Alkylreste sind.

In einer bevorzugten Ausführungsform weist der Metallkomplex die Formel (1) auf:

Mₓ[(L¹)ₐ(L²)_{b}(L³)_{c}X_{d}] (1)

wobei
die Liganden **L,** nämlich L¹, L² und L³, unabhängig voneinander ausgewählt sind aus Resten der Formel R¹-N₃-R², wobei R¹ und R² Kohlenwasserstoffreste sind,
   wobei mindestens bei L¹ die Reste R¹ und R² Alkylreste sind,
**X** unabhängig voneinander ausgewählt ist aus H, Halogen, CO und Kohlenwasserstoffliganden, insbesondere Alkylresten mit 1 bis 12 C-Atomen und aromatischen Kohlenwasserstoffen mit 5 bis 30 C-Atomen,
x eine ganze Zahl zwischen 1 und 4 ist, bevorzugt 1 oder 2,
a, b, c und d ganze Zahlen sind, wobei
   die Summe a + b + c + d mindestens x ist und nicht größer als 12 ist, bevorzugt nicht größer als 6, insbesondere nicht größer als 4 ist,
   a mindestens 1 ist, bevorzugt von 1 bis 6 ist, und dabei bevorzugt 1 oder 2 ist,
   b, c und d = 0 sein können, und bevorzugt jeweils 0, 1 oder 2 sind.
Dabei ist b insbesondere 0 oder 1, und c ist bevorzugt 0.

Wie dem Fachmann bekannt ist, ergibt sich das Verhältnis der Liganden zu dem Metall aus der Oxidationsstufe des Metalls. Dabei ist ein Ligand **L** der Formel R¹-N₃-R², wobei R¹ und R² Kohlenwasserstoffreste sind, einfach negativ geladen. Somit kann beispielsweise ein Metall der Oxidationsstufe II, wie Co(II), in der Formel (1) mit einem oder zwei Liganden **L** substituiert sein.

Allgemein ist es bevorzugt, dass die Komplexe möglichst homogen sind. Dann ist oft der Zerfallsprozess weniger komplex, und dadurch die Wahrscheinlichkeit niedriger, dass in der Gasphase und bei der Beschichtungsreaktion unerwünschte Nebenreaktionen auftreten. Insbesondere ist es bevorzugt, dass ein Komplex nur eine oder zwei verschiedene Typen von Liganden aufweist, beispielsweise nur einen Typ eines Liganden **L** und einen Typ **X,** oder nur zwei Typen des Liganden **L.** Besonders bevorzugt weist der Komplex insgesamt als Liganden **L** nur L¹ und L² auf. Dann ist c = 0.

Besonders bevorzugt weist der Komplex nur Liganden **L** auf, die L¹ sind. Dann ist b = 0 und c = 0. In einer bevorzugten Ausführungsform weist der Komplex dabei keinen Liganden **X** auf, so dass d = 0 ist. Bevorzugt sind dabei auch Ausführungsformen, bei denen c = d = 0 ist, oder bei denen b = c = d = 0 ist. Es wurde gefunden, dass solche Komplexe, wohl wegen der geringen strukturellen Komplexität, oft ein besonders gutes Verhalten bei Abscheidungsprozessen in der Gasphase zeigen.

In einer bevorzugten Ausführungsform ist der Metallkomplex homoleptisch. Dabei weist der homoleptische Komplex nur einen oder mehrere Liganden **L** auf. Der Begriff "homoleptisch" bedeutet, dass alle Liganden einer Verbindung identisch sind. Homoleptische Komplexe sind besonders bevorzugt, weil das Zerfallsverhalten in der Gasphase oft gleichförmiger und besser kontrollierbar ist als bei heteroleptischen Metallkomplexen, die verschiedene Liganden aufweisen. Erfindungsgemäß wurde gefunden, dass homoleptische Komplexe besonders gute Eigenschaften hinsichtlich Stabilität und Volatilität aufweisen, die für die Gasphasenabscheidung erforderlich ist. Dabei wurden insbesondere vorteilhafte Eigenschaften für homoleptische Komplexe von In, Co, Cu und La gefunden.

In einer weiteren bevorzugten Ausführungsform ist der Metallkomplex heteroleptisch. Das bedeutet, dass ein Metallkomplex zwei oder mehr verschiedene Liganden aufweisen kann, insbesondere zwei, drei oder vier verschiedene Liganden. Dabei können zwei oder mehr verschiedene Liganden **L** der Formel R¹-N₃-R² vorhanden sein. Alternativ können mindestens ein Ligand **L** der Formel R¹-N₃-R² und mindestens ein weiterer Ligand vorhanden sein. In einer bevorzugten Ausführungsform weist der heteroleptische Metallkomplex zwei oder drei verschiedene Liganden auf. Erfindungsgemäß wurde gefunden, dass beispielsweise heteroleptische Rutheniumkomplexe gut zugänglich sind und zur Abscheidung von Ru aus der Gasphase geeignet sind.

In einer bevorzugten Ausführungsform weist der Metallkomplex ausschließlich Liganden **L** der Formel R¹-N₃-R² auf.

In einer weiteren bevorzugten Ausführungsform weist der Metallkomplex zusätzlich weitere Liganden auf. Dabei sind bevorzugt keine weiteren Liganden vorhanden, welche die vorteilhaften Eigenschaften hinsichtlich Stabilität und Volatilität beeinträchtigen, oder die Abscheidung des reinen Metalls aus der Gasphase beeinträchtigen. Bevorzugt ist daher kein weiterer Ligand vorhanden, der ein reaktives Element aufweist, das insbesondere ausgewählt ist aus O, N, S oder P. Insbesondere ist bevorzugt kein weiterer Ligand mit einer Amingruppe vorhanden, wie Tetramethylethylendiamin (TMEDA). In einer Ausführungsform ist bevorzugt kein Ligand vorhanden, der ein Halogen enthält. In einer weiteren bevorzugten Ausführungsform ist kein Ligand vorhanden, das ein Strukturelement N-C-N oder N-Aryl aufweist.

In einer bevorzugten Ausführungsform weist der Metallkomplex ein Metallatom und einen bis vier Liganden der Formel R¹-N₃-R² auf, und dabei insbesondere eins, zwei, drei oder vier Liganden. Der Metallkomplex kann auch zwei, drei, vier oder mehr Metallatome aufweisen, wobei eine entsprechend höhere Anzahl an Liganden vorhanden ist. Die Stöchiometrien solcher Metallkomplexe sind allgemein bekannt. Der Rest R¹-N₃-R² ist dabei einfach negativ geladen.

Die Reste R¹ und R² sind Kohlenwasserstoffreste. Dies bedeutet, dass sie ausschließlich aus den Elementen Kohlenstoff und Wasserstoff bestehen. R¹ und R² können allgemein unabhängig voneinander ausgewählt sein.

In einer bevorzugten Ausführungsform ist R¹ = R². Dadurch kann die Verbindung insgesamt besonders homogen sein. Bei der Abscheidung von Metallen aus der Gasphase kann dies von Vorteil sein, weil der Zerfallsprozess weniger komplex ist und weniger Nebenreaktionen zu erwarten sind.

In einer bevorzugten Ausführungsform weisen R¹ und R² im Rahmen dieser Anmeldung unabhängig voneinander 1 bis 25 C-Atome, insbesondere 1 bis 20 C-Atome auf. Dabei ist es insbesondere bevorzugt, dass R¹ und R² unabhängig voneinander 1 bis 15 C-Atome, insbesondere 1 bis 12 C-Atome, aufweisen.

Die Reste R¹ und R² können unabhängig voneinander ausgewählt sein aus Alkyl, Alkenyl, Aryl und Araryl. In einer bevorzugten Ausführungsform sind R¹ und R² Alkylreste. Bevorzugt weist der Metallkomplex mindestens einen Liganden **L** der Formel R¹-N₃-R² auf, bei dem R¹ und R² Alkylreste sind. Bevorzugt sind bei allen Liganden **L** des Metallkomplexes R¹ und R² Alkylreste. Die Alkylreste können dabei verzweigt, zyklisch oder unverzweigt sein.

Im Rahmen dieser Anmeldung können die Alkylreste R¹ und R² beispielsweise 1 bis 20 C-Atome, insbesondere 1 bis 15 C-Atome oder 1 bis 12 C-Atome aufweisen. Besonders bevorzugt sind die Alkylreste relativ kurzkettig und weisen 1 bis 6 C-Atome, insbesondere 1 bis 4 C-Atome auf.

In einer bevorzugten Ausführungsform sind alle oder ein Teil der Reste R¹ und/oder R² des Metallkomplexes verzweigte und/oder zyklische Alkylreste. Bevorzugte verzweigte Reste sind dabei tert-Butyl und Isopropyl, insbesondere *tert*-Butyl. Bevorzugte zyklische Reste sind sperrige Reste, wie Adamantyl oder Cycloalkyl, insbesondere Cyclohexyl.

Besonders bevorzugt sind die Reste R¹ und R² ausgewählt aus Methyl, Ethyl, Propyl, Isopropyl, tert-Butyl und n-Propyl. Besonders bevorzugt sind die Reste R¹ und R² ausgewählt aus Methyl, Ethyl und *tert*-Butyl. In einer bevorzugten Ausführungsform sind R¹ und/oder R² *tert*-Butyl*.*

In einer bevorzugten Ausführungsform weist der Metallkomplex mindestens einen Liganden auf, der *tert*-Butyl-N₃-*tert*-Butyl oder *tert*-Butyl-N₃-Methyl ist. In einer bevorzugten Ausführungsform weist der Metallkomplex ausschließlich Liganden auf, die *tert*-Butyl-N₃-*tert*-Butyl und/oder *tert*-Butyl-N₃-Methyl sind. Dabei ist der Metallkomplex bevorzugt homoleptisch.

Der Metallkomplex kann einen weiteren Liganden **X** aufweisen. Der weitere Ligand **X** ist bevorzugt ausgewählt aus Halogen, H, CO und Kohlenwasserstoffliganden. Die Kohlenwasserstoffliganden sind insbesondere Alkylreste mit 1 bis 12 Kohlenstoffatomen, insbesondere 1 bis 4 Kohlenstoffatomen, besonders bevorzugt 1 oder 2 Kohlenstoffatomen. Besonders bevorzugt ist Methyl. Ebenfalls bevorzugt als Liganden **X** sind aromatische Kohlenwasserstoffe mit 5 bis 30 C-Atomen, insbesondere 6 bis 12 C-Atomen, wie Penten, Benzen oder substituiertes Benzen, wie Cymen oder verschiedene Di- oder Trialkybenzene. Die aromatischen Reste X können neutral oder negativ geladen sein. Es ist bekannt, dass solche aromatischen Liganden Metallkomplexe stabilisieren können, beispielsweise Komplexe von Ru. Außerdem können die Liganden Alkenylliganden sein, insbesondere mit 1 bis 12 C-Atomen. Das Halogen ist bevorzugt Cl.

Das Metall M kann ein Metall der Haupt- und Nebengruppen des Periodensystems der Elemente sein (ausgenommen Alkalimetalle). Erfindungsgemäß sind für die Abscheidung des Metalls oder einer Verbindung des Metalls aus der Gasphase überaschenderweise Metallkomplexe verwendbar, die eine große Bandbreite von Metallen aufweisen können.

Es wurde gefunden, dass die Verwendung besonders effizient ist, wenn Metallkomplexe von Metalle der Gruppen 8, 9 und 10 des Periodensystems (insbesondere Fe, Ru, Os, Co, Rh, Ir, Ni, Pd, Pt), der Gruppe 13 (Al, Ga, In, Tl), der Gruppe 10 (Cu, Ag, Au), der Gruppe 12 (insbesondere Zn; oder Cd oder Hg), der Lanthanoiden (La, Ce, Pr, Nd, etc.) oder der Actinoiden (Ac, Th, Pa, U, etc.) eingesetzt werden. Bevorzugt weist das Metall in dem Komplex die Oxidationsstufe 1, 2 oder 3 auf.

In einer bevorzugten Ausführungsform ist das Metall M ausgewählt aus In, Co, Ru, Cu, Al, Ga, Tl und La. Komplexe dieser Metalle mit Triazenidoliganden sind mit dem erfindungsgemäßen Verfahren auf relativ einfache Weise zugänglich. Es wurde gefunden, dass diese Metallkomplexe in hohem Maße zur Abscheidung der Metalle aus der Gasphase geeignet sind.

Besonders bevorzugt werden Metalle ausgewählt aus Al, In, Co, Cu und La eingesetzt. Es wurde gefunden, dass Metallkomplexe dieser Metalle besonders gut zugänglich sind und besonders vorteilhafte Eigenschaften bei der Gasphasenabscheidung zeigen.

In einer bevorzugten Ausführungsform ist das Metall M ausgewählt aus In, Co, Cu und Ru. Es besteht im Stand der Technik insbesondere ein Bedarf nach Vorläuferverbindungen zur Abscheidung solcher Metalle aus der Gasphase.

In einer bevorzugten Ausführungsform ist das Metall M = Co. Es wurde gefunden, dass Co-Komplexe besonders gut sublimierbar sind, wobei niedermolekulare Komplexe dabei bei relativ niedriger Temperatur und nahezu rückstandsfrei verdampft werden können.

In einer bevorzugten Ausführungsform ist das Metall M = In. Es wurde gefunden, dass In-Komplexe besonders gut sublimierbar oder verdampfbar sind, wobei bei relativ niedriger Temperatur ein großer Anteil verdampft werden kann. In einer bevorzugten Ausführungsform ist der In-Komplex bei 25°C flüssig. So wurde gefunden, dass die Verbindung In(dbt)Me₂ (Formel 9a, Beispiel 8) bei 25°C flüssig ist. In-Komplexe, die leicht in die Gasphase überführbar sind, sind besonders vorteilhaft, weil im Stand der Technik im Allgemeinen In-Alkyl Verbindungen, wie Trimethylindium, eingesetzt werden, die bei Raumtemperatur Feststoffe sind und zu einer unkontrollierten, teilweise explosionsartigen autokatalytischen Zersetzung neigen.

In einer bevorzugten Ausführungsform ist das Metall M = Ru. Solche Komplexe sind vorteilhaft, da im Stand der Technik nur wenige und verbesserungsbedürftige Vorläuferverbindungen zur Verfügung stehen, um Ru bei relativ niedriger Temperatur in die Gasphase zu überführen und abzuscheiden. In einer bevorzugten Ausführungsform ist der Ru-Komplex bei 25°C flüssig. So wurde gefunden, dass die Verbindungen der Formeln (14) und (16) (Beispiele 14 und 16) bei 25°C flüssig sind, während weitere Ru-Komplexe sehr niedrige Schmelzpunkte von weniger als 70°C aufweisen. Flüssige und feste Ru-Komplexe, die leicht in die Gasphase überführbar sind, sind besonders vorteilhaft, weil im Stand der Technik keine Ru-Verbindungen zur Verfügung stehen, die effizient in die Gasphase überführt und bei solchen Verfahren eingesetzt werden können.

In einer bevorzugten Ausführungsform ist das Metall M = Cu. Es wurde gefunden, dass Cu(I)-Komplexe der Zusammensetzung [Cu(R¹-N₃-R²)]ₙ je nach Größe der Reste R¹ bzw. R² als Dimer (n = 2, R¹ = R² = *tert*-Butyl) oder als Tetramer (n = 4, R¹ = *tert*-Butyl, R² = Methyl) im kristallinen Festkörper vorliegen. Die Bildung derartiger Assoziate ist typisch für viele Metallverbindungen. Dennoch sind beide Verbindungen sehr gut sublimierbar, wobei niedermolekulare Komplexe bei relativ niedriger Temperatur und nahezu rückstandsfrei in die Gasphase verdampft werden können.

In einer bevorzugten Ausführungsform ist das Metall M = La. Solche Komplexe sind vorteilhaft, da im Stand der Technik nur wenige und verbesserungsbedürftige Vorläuferverbindungen zur Verfügung stehen, um Lanthan oder andere Lanthanoide, die sehr hohe Molekulargewichte aufweisen, bei relativ niedriger Temperatur in die Gasphase zu überführen und abzuscheiden.

In einer bevorzugten Ausführungsform weist der Metallkomplex die Formel (2) auf:

M[(L¹)ₐX_{d}] (2)

wobei
L¹ die Formel R¹-N₃-R² aufweist, wobei R¹ und R² Alkylreste mit 1 bis 12 C-Atomen sind,
X ausgewählt ist aus H, Halogen, CO und Alkyl mit 1 bis 6 C-Atomen,
a = 2 oder 3 ist,
d = 0 oder 1 ist, und
M ausgewählt ist aus In, Co, Cu, Al, Ga, Tl und La.

In einer bevorzugten Ausführungsform weist der Metallkomplex eine der Formeln (3) bis (5) auf:

M[(L¹)₂X] (3)

wobei
L¹ die Formel R¹-N₃-R² aufweist, wobei R¹ und R² Alkylreste mit 1 bis 12 C-Atomen sind,
X ausgewählt ist aus H und Alkyl mit 1 bis 6 C-Atomen, und
M = Al oder Ga ist;

M[(L¹)₃] (4)

wobei
L¹ die Formel R¹-N₃-R² aufweist, wobei R¹ und R² Alkylreste mit 1 bis 12 C-Atomen sind, und
M = In, Tl oder La ist;

Mₓ(L¹)ₐ (5)

wobei
L¹ die Formel R¹-N₃-R² aufweist, wobei R¹ und R² Alkylreste mit 1 bis 12 C-Atomen sind,
x eine ganze Zahl zwischen 1 und 4 ist, und
a eine ganze Zahl zwischen 2 und 8 ist, mit der Maßgabe, dass a/x = 1 oder 2 ist, und
M = Co oder Cu ist.
Dabei ist insbesondere das Metall Co, wenn a/x = 2 ist, und das Metall Cu, wenn a/x = 1 ist.

In einer bevorzugten Ausführungsform weist der Metallkomplex die Formel (6) aufweist:

(Ru[(L¹)X¹X²] (6)

wobei
L¹ die Formel R¹-N₃-R² aufweist, wobei R¹ und R² Alkylreste mit 1 bis 12 C-Atomen sind,
X¹ ein aromatischer Kohlenwasserstoffligand mit 5 bis 30 C-Atomen ist, und
X² ausgewählt ist aus keinem Rest, H, Halogen, CO und Alkyl mit 1 bis 6 C-Atomen.

Es wurde gefunden, dass die Stabilität und Volatilität der Ru-Komplexe geringer sein kann als die anderer Metalle. Ungeachtet dessen lässt sich mit den Ru-Komplexen elementare Ru aus der Gasphase abscheiden. Dies ist vorteilhaft, weil im Stand der Technik ein Bedarf nach Ru-Verbindungen besteht, die als Vorläuferverbindungen in solchen Verfahren geeignet sind. Die Ru-Komplexe können beispielsweise verwendet werden, um Ru als Dotierungsmetall, als Liner-Metall oder als Filling-Metall in Materialien für elektronische Anwendungen einzubauen.

In einer bevorzugten Ausführungsform ist der Metallkomplex bei 25°C und Normaldruck (1013 mbar) flüssig. Flüssigkeiten sind besonders gut für solche Verfahren geeignet, weil der Übergang in die Gasphase bereits bei niedriger Temperatur erfolgt, und weil Flüssigkeiten besonders gut handhabbar sind. In einer bevorzugten Ausführungsform ist der Metallkomplex bei 25°C und Normaldruck fest und weist eine relativ niedrige Verdampfungstemperatur auf.

Bevorzugt weist der Metallkomplex eine Sublimations- oder Verdampfungstemperatur bei Normaldruck auf, die kleiner als 120°C ist, insbesondere kleiner als 100°C.

Bevorzugt erfolgt der Übergang in die Gasphase durch Verdampfung oder Sublimation bei einer Temperatur, die kleiner als 120°C ist, insbesondere kleiner als 100°C. Die Verdampfung oder Sublimation erfolgt dabei bevorzugt bei reduziertem Druck im Bereich von 10⁻³ bis 900 mbar, bevorzugt im Bereich von 10⁻² bis 1 mbar, und insbesondere bei 10⁻² mbar.

Allgemein wird der Druck bei dem erfindungsgemäßen Verfahren bevorzugt in Bereichen eingestellt, die für solche Verfahren üblich sind. Somit werden das Verfahren und die Verwendung bevorzugt bei einem Druck im Bereich von 10⁻³ bis 900 mbar, und besonders bevorzugt im Bereich von 10⁻² bis 1 mbar durchgeführt, und insbesondere bei 10⁻² mbar. Im Rahmen dieser Anmeldung werden Parameter bei der Reaktion in der Gasphase, wie die Verdampfungs-, Sublimations- oder Zersetzungstemperaturen, oder Temperaturen, bei denen Verbindungen stabil sind, bevorzugt bei den reduzierten Drücken gemessen, die bei solchen Reaktionen eingestellt werden, und dabei insbesondere bei einem Druck von 10⁻² mbar.

In einer bevorzugten Ausführungsform ist der Metallkomplex bei Normaldruck und/oder bei reduziertem Druck in der Gasphase sublimierbar oder verdampfbar, ohne dass eine Zersetzung (ein Zerfall) erfolgt. Die bedeutet beispielsweise, dass bis zu 90% Gew.%, bis zu 95% Gew.% oder bis zu 98% Gew.% in die Gasphase überführt werden können, ohne dass eine Zersetzung erfolgt. Der Zerfall lässt sich nach üblichen Methoden verfolgen, insbesondere SDTA (Simultaneous differential thermal analysis).

Bevorzugt ist der Metallkomplex bei Normaldruck, aber insbesondere bei reduziertem Druck in der Gasphase bei 100°C, besonders bevorzugt bei 120°C oder 150°C, thermisch stabil. Dies bedeutet, dass bei einer solchen Temperatur noch keine wesentliche Zersetzung einsetzt, oder dass zumindest nicht mehr als 5 Gew.%, insbesondere nicht mehr als 2 Gew.% oder nicht mehr als 1 Gew.% zersetzt werden. Bevorzugt geht der Metallkomplex unterhalb dieser Temperatur in die Gasphase über. Dabei liegt der reduzierte Druck im Bereich von 10⁻³ bis 900 mbar, bevorzugt im Bereich von 10⁻² bis 1 mbar, und insbesondere bei 10⁻² mbar.

In einer bevorzugten Ausführungsform weist der Metallkomplex ein relativ niedriges Molekulargewicht auf. Relativ leichte Komplexe können oft besser in die Gasphase überführt werden. Die im Rahmen dieser Anmeldung beschriebenen Metallkomplexe können in vorteilhafter Weise mit Liganden hergestellt werden, die ein relativ geringes Molekulargewicht aufweisen. Das Molekulargewicht des Metallkomplexes ist bevorzugt kleiner als 600 g/Mol, besonders bevorzugt kleiner als 450 g/Mol, und ganz besonders bevorzugt kleiner als 350 g/Mol.

Gegenstand der Erfindung ist auch ein Verfahren zur Herstellung von beschichteten Substraten, umfassend die Schritte:
(a) Bereitstellen eines wie oben beschriebenen Metallkomplexes, und
(b) Abscheiden des Metalls oder einer Verbindung des Metalls auf der Oberfläche des Substrats durch metallorganische Gasphasenabscheidung.

In einer bevorzugten Ausführungsform ist das Verfahren eine metallorganische chemische Gasphasenabscheidung (MOCVD). Die metallorganische Gasphasenabscheidung (MOCVD, "Metallo-Organic Chemical Vapor Deposition") ist ein Beschichtungsverfahren aus der Gruppe der chemischen Gasphasenabscheidung (CVD), bei dem die Abscheidung einer festen Schicht auf ein Substrat aus der chemischen Dampfphase unter Einsatz einer metallorganischen Vorläuferverbindung (Precursor) erfolgt.

In einer bevorzugten Ausführungsform ist das Verfahren eine metallorganische chemische Gasphasenepitaxie (MOVPE; für "Metal Organic Vapor Phase Epitaxy", auch "Organometallic Vapor Phase Epitaxy", OMVPE). Während durch MOCVD jede Abscheidung auf einem Substrat möglich ist, ist MOVPE ein Epitaxie-Verfahren und betrifft somit das kristalline Wachstum auf einem kristallinen Substrat. Die Verfahren, insbesondere MOVPE, werden insbesondere zur Abscheidung von halbleitenden Materialien verwendet.

Mit den erfindungsgemäßen Verwendungen und Verfahren wird das Metall oder eine Verbindung des Metalls aus der Gasphase abgeschieden. Dabei erfolgt die Abscheidung auf einem Substrat. Die metallhaltige Abscheidung wird dabei im Allgemeinen als Schicht oder Beschichtung bezeichnet. Die Verbindung des Metalls ist bevorzugt ausgewählt aus Halbleiterverbindungen, Legierungen, Nitriden, Phosphiden, Arseniden und Siliziden. Solche Verbindungen werden nach bekannten Verfahren durch Zusatz von weiteren Verbindungen in der Gasphase erhalten, die mit dem Metall unter den Bedingungen in der Vorrichtung reagieren können. Für eine Übersicht wird ebenfalls auf die oben zitierte Publikation von J. Zilko verwiesen.

Verfahren der metallorganischen Gasphasenabscheidung und der metallorganischen Gasphasenepitaxie werden bevorzugt wie nachfolgend beschrieben durchgeführt. Die Herstellung der metallhaltigen Schichten erfolgt in einer Reaktionskammer. In dieser liegt ein zu beschichtendes Substrat vor, das auf eine erhöhte Temperatur geheizt wird. Ein Gasstrom mit dem Metallkomplex als Vorläuferverbindung und üblicherweise einem Trägergas wird in die Reaktionskammer eingeführt, wo die Vorläuferverbindung in der Gasphase vorzerlegt und radikalische Gruppen sich auf dem Substrat anlagern. Thermisch aktiviert haben radikalische Gruppen eine gewisse Bewegungsfreiheit auf dem Substrat, bis sich das Metallatom an geeigneter Stelle in die Schicht einbaut. Der organische Rest wird mit elementarem Wasserstoff gesättigt, so dass eine volatile organische Verbindung entsteht. Das Restgas wird aus der Reaktionskammer abgeführt. Es ist bevorzugt, dass der Metallkomplex vor Einführung in die Reaktionskammer in die Gasphase überführt wird. In einer weiteren Ausführungsform ist es auch denkbar, eine Flüssigkeit, die den Metallkomplex enthält, erst bei Einführung in die Reaktionskammer zu verdampfen.

Überraschenderweise wurde gefunden, dass die im Rahmen dieser Anmeldung beschriebenen Metallkomplexe in hohem Maße als Vorläuferverbindungen zur Abscheidung von Metallen aus der Gasphase geeignet sind. Sie vereinen dabei überraschenderweise mehrere vorteilhafte Eigenschaften, die für solche Verfahren erforderlich sind. Sie sind in hohem Maße stabil, volatil und können so eingesetzt werden, dass bei der Abscheidung des Metalls oder einer Verbindung des Metalls auf einem Substrat nahezu keine Verunreinigungen entstehen.

Bevorzugt wird der Metallkomplex bei dem Verfahren sublimiert oder verdampft, ohne dass eine Zersetzung erfolgt. Während bei der Sublimation ein Feststoff vorgelegt wird, wird bei der Verdampfung eine Flüssigkeit oder eine Lösung vorgelegt. Bevorzugt erfolgt also während der Überführung des Metallkomplexes in die Gasphase kein Zerfall oder kein wesentlicher Zerfall. Der Zerfallsprozess lässt sich nach üblichen Methoden verfolgen, insbesondere SDTA (Simultaneous differential thermal analysis).

Die relevanten Eigenschaften eines Metallkomplexes bei Erhöhung der Temperatur lassen sich durch Thermogravimetrische Analyse (TGA) bestimmen, die bei normalem Druck (1013 mbar) oder auch bei reduziertem Druck durchführbar ist. Dabei ist es bevorzugt, dass die Kurve Menge/Temperatur (bei gleichmäßiger T-Erhöhung, z. B. 5 oder 10 K/min) einen gleichmäßigen Verlauf zeigt. Eine Sublimation liegt dabei im Allgemeinen vor, wenn bei oder ab einer Sublimationstemperatur eine schnelle Gewichtsabnahme erfolgt, und dabei kein oder nur wenig Rückstand verbleibt. Bevorzugt zeigt die TGA-Kurve keine wesentlichen Unregelmäßigkeiten, wie Höcker oder Dellen.

Bevorzugt erfolgt die Überführung des Metallkomplexes in die Gasphase möglichst vollständig. Dabei ist es bevorzugt, dass bei der TGA bei Normaldruck und/oder reduziertem Druck ein möglichst geringer Rückstand verbleibt, beispielsweise weniger als 20 Gew.-%, weniger als 10 Gew.%, weniger als 5 Gew.-%, oder insbesondere weniger als 3 Gew.%, bezogen auf die eingesetzte Menge des Metallkomplexes. Der Rückstand entspricht dem Plateauwert, an dass sich das das Gewicht des Rückstandes bei weiterer T-Erhöhung auf beispielsweise bis zu 500°C, 600°C oder 700°C annähert. Wenn das Gewicht des Rückstandes (deutlich) unterhalb dem Gewicht des Metalls in der vorgelegten Verbindung liegt, deutet dies auf eine Sublimation oder Verdampfung der Verbindung hin. Bevorzugt wird bei der TGA mindestens 50 Gew.%, insbesondere mindestens 80 Gew.% oder mindestens 90 Gew.% des Metalls in die Gasphase überführt. Allgemein ist es aus Effizienzgründen vorteilhaft, wenn das Metall quantitativ verdampft wird.

Es wurde gefunden, dass die erfindungsgemäßen Metallkomplexe relativ stabil sind. Sie sind bevorzugt bei Raumtemperatur stabil und dadurch beliebig lange haltbar. In einer bevorzugten Ausführungsform ist der Metallkomplex bei Temperaturen bis zu 100°C thermisch stabil.

Bevorzugt erfolgt bei der TGA bei Normaldruck ein Massenverlust von 3% erst bei einer Temperatur oberhalb 80°C, bevorzugt oberhalb 100°C, und besonders bevorzugt oberhalb 120°C. Bevorzugt erfolgt der Massenverlust von 3% bei einer Temperatur zwischen 80°C und 220°C, insbesondere zwischen 80°C und 160°C.

Bevorzugt zerfällt der Metallkomplex unter Bedingungen der Gasphasenabscheidung, die üblicherweise bei reduziertem Druck durchgeführt wird, bei Temperaturen zwischen 100°C und 400°C, insbesondere zwischen 120°C und 300°C, oder zwischen 150°C und 250°C. Bevorzugt liegt der Komplex bei diesen Temperaturen in der Gasphase vor. Dabei liegt der reduzierte Druck im Bereich von 10⁻³ bis 900 mbar, bevorzugt im Bereich von 10⁻² bis 1 mbar, und insbesondere bei 10⁻² mbar. Der Zerfall erfolgt oft in einer exothermen Reaktion. Der Zerfall in diesem Bereich ist bevorzugt vollständig.

Bevorzugt wird der Metallkomplex erst nach Überführung in die Gasphase zersetzt. Bevorzugt erfolgt somit die Zersetzung bei einer Temperatur, die oberhalb der Sublimationstemperatur oder Verdampfungstemperatur liegt. Bevorzugt liegt die Zersetzungstemperatur bei nicht mehr als 200°C, nicht mehr als 100°C, oder bevorzugt nicht mehr als 50°C oberhalb der Sublimationstemperatur oder Verdampfungstemperatur, insbesondere bei reduziertem Druck wie oben angegeben. Bevorzugt ist der Metallkomplex bei dieser Zersetzungstemperatur vollständig, oder zu 50%, zersetzt. Wenn die Zersetzung bei einer Temperatur erfolgt, die nicht allzu weit oberhalb der Sublimation- oder Verdampfungstemperatur liegt, kann die Abscheidung in der Gasphase oft besonders effizient durchführbar sein.

Bevorzugt wird bei der Abscheidung aus der Gasphase das elementare Metall oder die gewünschte Verbindung des Metalls in Reinform erhalten. Dies bedeutet, dass keine oder vernachlässigbare Verunreinigungen bestimmt werden können, beispielsweise von weniger als 500 ppb, weniger als 100 ppb oder weniger als 10 ppb. Die Reinheit des abgeschiedenen Metalls oder der metallhaltigen Verbindung kann mittels üblicher Methoden bestimmt werden, beispielsweise durch Sekundärionenmassenspektrometrie.

In einer weiteren bevorzugten Ausführungsform enthält die Beschichtung mindestens eine weitere atomare Komponente, insbesondere mindestens ein weiteres Metall, oder ein Element, ausgewählt aus Si, N, P oder As. So ist es nach bekannten Methoden möglich, weitere Metalle oder Elemente in die Beschichtung einzuarbeiten, um entsprechende Legierungen oder Verbindungen zu erhalten.

Gegenstand der Erfindung ist auch ein Verfahren zur Herstellung des erfindungsgemäßen oder erfindungsgemäß verwendbaren Metallkomplexes, umfassend die Schritte
(A) Bereitstellen einer Verbindung der Formel R¹-(N₃)A-R², wobei A ausgewählt ist aus H oder einem Alkalimetall, insbesondere Li, Na oder K, besonders bevorzugt Li, und
(B) in Kontakt bringen mit einer Verbindung des Metalls.

Die Verbindung der Formel R¹-(N₃)A-R² reagiert dann mit der Verbindung des Metalls zu dem Metallkomplex. Bevorzugt erfolgt diese Reaktion in einem einzigen Schritt. Dabei ist A bevorzugt Alkali, insbesondere Li. Die Metallkomplexe und insbesondere die Reste R¹, R² und A sind dabei ausgewählt wie oben beschrieben.

Bevorzugt ist dabei die in Schritt (B) eingesetzte Verbindung des Metalls ein Metallsalz, eine metallorganische Verbindung oder ein anderer Metallkomplex des Metalls. Dabei weist der andere Metallkomplex bevorzugt keinen Triazenidoliganden auf, insbesondere keinen Liganden L der Formel R¹-N₃-R², bei dem R¹ und R² Kohlenwasserstoffreste sind.

In einer bevorzugten Ausführungsform umfasst das Verfahren zur Herstellung der Metallkomplexe vor Schritt (A) den Schritt des Herstellens der Verbindung der Formel R¹-(N₃)A-R² aus den Verbindungen R¹-N₃ und AR², wobei A ein Alkalimetall ist. Dabei wird das gesamte Verfahren bevorzugt in einem einzigen Reaktionsgemisch durchgeführt (als "Eintopfreaktion").

Gegenstand der Erfindung ist auch ein Verfahren zur Herstellung einer Verbindung der Formel R¹-(N₃)A-R² in einem Reaktionsgemisch, das die Verbindungen R¹-N₃ und AR² enthält, wobei A ein Alkalimetall ist, bevorzugt Li. Die Reste R¹, R² und A sind dabei ausgewählt wie oben beschrieben.

Die Herstellung einer Verbindung der Formel R¹-(N₃)A-R² erfolgt bevorzugt in einem inerten Lösungsmittel, insbesondere einem Kohlenwasserstoff, wie Pentan oder Hexan.

In einer Ausführungsform wird die Verbindung der Formel R¹-(N₃)A-R² nach der Reaktion aufgereinigt, indem die weiteren Komponenten aus dem Reaktionsgemisch verdampft werden (Methode A). Wenn A ein Alkalimetall ist und sich in Diethylether befindet, fällt das Produkt als festes und reines Diethyletheraddukt (Alkalisalz) aus und kann durch Filtration abgetrennt werden (Methode B). Das Diethyletheraddukt kann im Vakuum komplett von Ether befreit werden oder es kann, falls gewünscht, durch Hydrolyse mit Wasser in die korrespondierende Neutralverbindung (mit A = H) überführt werden.

Erfindungsgemäß wurde gefunden, dass mit dem erfindungsgemäßen Verfahren Metallkomplexe, sowie die Verbindungen der Formel R¹-(N₃)A-R², die wesentliche Zwischenprodukte für die Komplexsynthese sind, auf relativ einfache Weise, effizient und in hoher Ausbeute herstellbar sind. Bei der Herstellung des Metallkomplexes wird dabei von einer Verbindung der Formel R¹-(N₃)A-R² ausgegangen. Dabei kann A ein Wasserstoffrest H sein. Die Verbindung ist dann insgesamt neutral. Es wurde gefunden, dass mit verschiedenen Metallsalzen eine effiziente Metallkomplexbildung unter relativ einfachen Bedingungen stattfindet. So sind beispielsweise Metallkomplexe von Al, Ga und In ausgehend von diesen Verbindungen erhältlich.

Auch wurde gefunden, dass viele Metallkomplexe zugänglich sind, wenn die Verbindung der Formel R¹-(N₃)A-R² ein Alkalimetallsalz ist. Dabei ist A insbesondere Li, Na oder K, und besonders bevorzugt Li. Solche Verbindungen wurden erfindungsgemäß beispielsweise eingesetzt, um auf einfache Weise und mit hoher Ausbeute Salze von Co, Cu oder Ru herzustellen.

Die Verbindungen der Formel R¹-(N₃)A-R², wobei A = H oder ein Alkalimetall ist, sind auf einfache Weise durch Reaktion der Vorläuferverbindungen R¹-N₃ und A-R² erhältlich, wobei A bevorzugt ein Alkalimetall ist. Die Reaktion findet bevorzugt in einem organischen aprotischen Lösungsmittel statt, insbesondere in Kohlenwasserstoffen, besonders bevorzugt in Pentan oder Hexan. Dabei wird eine Alkalialkylverbindung, insbesondere eine Organo-Lithiumverbindung, bevorzugt langsam zugetropft. Bevorzugt wird das Reaktionsgemisch zunächst bei niedriger Temperatur, beispielsweise <15°C, belassen und anschließend auf Raumtemperatur erwärmt.

Bei der Reaktion zu dem Metallkomplex wird die so erhaltene Verbindung der Formel R¹-(N₃)A-R², oder die gleiche Verbindung aus anderer Herkunft, in Kontakt mit einer Verbindung des Metalls mit ausreichender Reaktivität gebracht, wie einem Metallsalz oder einer organometallischen Verbindung des Metalls, insbesondere mit einem Metallhalogenid, wie CoCl₂ oder CuCI, oder einer organometallischen Verbindung, wie AlMe₃ oder InMe₃, oder einem Metallhydrid, wie GaH₃ (OEt₂), oder einem Vorläufermetallkomplex, wie La(HMDS)₃ (HMDS = Hexamethyldisilazan) oder [RuCl₂(p-Cymen)]₂. Dabei wurde gefunden, dass beispielsweise im Falle von Kobalt bereits eine Reaktion mit einem einfachen Salz ausreicht, während bei Elementen der dritten Hauptgruppe metallorganische Vorläuferverbindungen bevorzugt sind. Um Ruthenium oder Lanthan in erfindungsgemäße Metallkomplexe zu überführen, wird bevorzugt von anderen Metallkomplexen ausgegangen. In allen Fällen wurde gefunden, dass ausgehend von Verbindungen der Formel R¹-(N₃)A-R² stabile Komplexe mit den entsprechenden Liganden in hohen Ausbeuten erhalten werden können.

Ohne an eine Theorie gebunden zu sein, wird vermutet, dass die relativ schwache N-N-Einfachbindung in den Metallkomplexen zu einer relativ leichten thermischen Zersetzbarkeit bei relativ niedrigen Temperaturen führt. Dabei wird angenommen, dass N₂ als stabiles Zersetzungsprodukt entsteht, während weitere stickstoffhaltige Alkylreste als molekulare flüchtige Verbindungen gebildet werden. Alle Reaktionsprodukte sind nicht oder wenig reaktiv und sicher in der Handhabung, so dass das Verfahren auf relativ einfache Weise und mit hoher Ausbeute durchführbar ist.

Vorteilhaft ist auch, dass die Liganden, bei denen R¹ oder R² beispielsweise Methyl oder tert-Butyl ist, relativ niedrige Molekulargewichte aufweisen. Dies führt zu einer relativ guten Verdampfbarkeit oder sogar Sublimierbarkeit bei relativ niedrigen Temperaturen. Die Anwendung gerade dieser beiden Liganden ist, wie überraschend gefunden wurde, von besonderer Bedeutung für die Bereitstellung hochgradig flüchtiger Metallverbindungen für ALD und MOCVD. Die Synthese über das leicht zugängliche und vor allem technologisch sicher handhabbare *tert*-Butylazid birgt erhebliche Vorteile gegenüber etablierten 1,3-Diazaallyl-Systemen wie N,N'-Dialkylacetamido- oder N,N'-Dialkyl-N"-dialkylguanidato-Ligandensystemen. Die Zerfallstemperaturen der im folgenden beschriebenen Triazenido-Komplexe unter ALD- und CVD-Bedingungen ist niedriger, der unerwünschte Kohlenstoffeinbau weniger prägnant als in Amidinato- und Guanidinato-Komplexen.

Bevorzugt wird bei Schritt (B) eine metallorganische Verbindung eingesetzt, insbesondere eine Organolithiumverbindung, insbesondere Alkyllithium mit 1 bis 6 C-Atomen, wie Methyllithium. Bevorzugt wird die Verbindung in Ether gelöst eingesetzt. Dabei wird als Lösungsmittel bevorzugt Pentan eingesetzt. Bevorzugt wird in Schritt (B) die Verbindung des Metalls zu der Verbindung aus Schritt (A) zugegeben, insbesondere kontinuierlich, wie durch Zutropfen.

Besonders vorteilhaft ist es erfindungsgemäß, wenn als Zwischenprodukt ein Salz des Liganden eingesetzt wird, insbesondere das Lithiumsalz. Bei der Herstellung solcher organischen Salze ist die Auswahl des Lösungsmittels von Bedeutung. So fällt das Lithiumsalz von Methyl-*tert*-Butyltriazen in Pentan als Lösungsmittel aus, wenn als Edukt Methyllithium in Ether gelöst eingesetzt wird. Das Lithiumsalz kann anschließend durch Filtration isoliert werden. Dagegen ist das Lithiumsalz von Di-*tert*-Butyltriazen in Pentan löslich. Es kann nach Ende der Reaktion durch Eindampfen der klaren Reaktionslösung isoliert werden. Bei beiden Synthesen fallen keine Nebenprodukte an und die Ausbeuten liegen bei über 80%. Falls gewünscht kann der Neutralligand Di-*tert*-Butyltriazen ohne weiteres durch wässrige Aufarbeitung des Reaktionsgemisches erhalten werden.

Die Lithiumsalze können vollständig getrocknet und weiterverarbeitet werden. Sie sind in unpolaren Lösungsmitteln, wie Pentan oder Toluol, löslich, aber auch in anderen gängigen Lösungsmitteln, wie Diethylether oder THF. Sie können daher in solchen Lösungsmitteln ohne weiteres in Folgereaktionen zur Herstellung der Metallkomplexe eingesetzt werden. Auch die entsprechenden Neutralliganden mit A = H, bei denen es sich um destillierbare Flüssigkeiten handelt, können in allen gängigen Lösungsmitteln für Folgereaktionen eingesetzt werden. Die erfindungsgemäßen Verfahren und die Zwischenprodukte ermöglichen daher eine einfache und effiziente Herstellung von Metallkomplexen in hohen Ausbeuten.

Ausgehend von den Verbindungen der Formel R¹-(N₃)A-R² sind so verschiedene Komplexverbindungen über die Routen der Salz-, Alkan- (insbesondere Methan), Wasserstoff- oder Amin-Eliminierung zugänglich. Die beiden Triazenidoliganden sind dabei in der Lage, Hauptgruppenelemente der dritten oder fünften Hauptgruppe, wie Gallium, Indium oder Antimon, in homoleptischen oder heteroleptischen Verbindungen zu komplexieren. Auch die Herstellung von Komplexen mit Nebengruppenelementen, wie Co, Cu, Ru oder La, ist ohne weiteres möglich.

Wegen der einfachen Herstellung der Zwischenprodukte und Metallkomplexe, die beispielsweise in einem einzigen Reaktionsansatz erfolgen kann, und da keine problematischen Nebenprodukte auftreten, kann das Verfahren ohne weiteres hochskaliert werden und beispielsweise im Industriemaßstab durchgeführt werden. Die Metallkomplexe werden bei üblichem Erwärmen in einer Vorrichtung zur Gasphasenabscheidung, insbesondere einem "Bubbler"-Vorratsgefäß, bei Erwärmung auf Temperaturen von beispielsweise 100°C nicht zersetzt. Sie zerfallen dann, bevorzugt erst nach Übergang in die Gasphase, bei Temperaturen im Bereich von 100°C bis 400°C.

Gegenstand der Erfindung ist auch ein Metallkomplex die Formel (1):

Mₓ[(L¹)ₐ(L²)_{b}(L³)_{c}X_{d}] (1)

wobei
die Liganden **L,** nämlich L¹, L² und L³, unabhängig voneinander ausgewählt sind aus Resten der Formel R¹-N₃-R², wobei R¹ und R² Kohlenwasserstoffreste sind,
   wobei mindestens bei L¹ die Reste R¹ und R² Alkylreste sind,
**X** unabhängig voneinander ausgewählt ist aus H, Halogen, CO und Kohlenwasserstoffliganden, insbesondere Alkylresten mit 1 bis 12 Kohlenstoffatomen und aromatischen Kohlenwasserstoffen mit 5 bis 30 C-Atomen,
x eine ganze Zahl zwischen 1 und 4 ist, bevorzugt 1 oder 2,
a, b, c und d ganze Zahlen sind, wobei
   die Summe a + b + c + d mindestens x ist und nicht größer als 12 ist, bevorzugt nicht größer als 6, insbesondere nicht größer als 4 ist,
   a mindestens 1 ist, bevorzugt von 1 bis 6 ist, und dabei bevorzugt 1 oder 2 ist,
b, c und d = 0 sein können, und bevorzugt jeweils 0, 1 oder 2 sind,
wobei mindestens eine der folgenden Bedingungen (i) bis (ii) erfüllt ist:
   (i) M ist ausgewählt aus Metallen der VIII. Nebengruppe und den Lanthaniden des Periodensystems der Elemente, insbesondere Ru, Co und La,
   (ii) mindestens ein Ligand **L** weist mindestens einen Rest R¹ oder R² auf, der *tert-*Butyl ist.

Allgemein sind die Metallkomplexe solche, die im Rahmen dieser Anmeldung für die Verwendungen und Verfahren beschrieben werden. Sie weisen daher insbesondere Metalle, Liganden und Reste, also insbesondere M, L¹, L², L³, R¹, R², X, x, a, b, c, d, auf, die ausgewählt sind wie weiter oben beschrieben. Bevorzugte Metallkomplexe sind insbesondere solche der Formeln M(L¹)₂, M(L¹)₃, M₂(L¹)₄ und M₂(L¹)₆. Allgemein ist x bevorzugt eine Zahl zwischen 1 und 2 und a + b + c + d bevorzugt eine Zahl zwischen 2 und 6.

In einer bevorzugten Ausführungsform weist der Metallkomplex eine der Formeln (7) bis (20) auf:

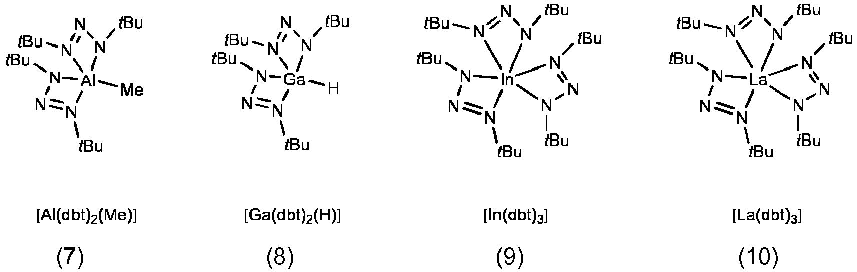

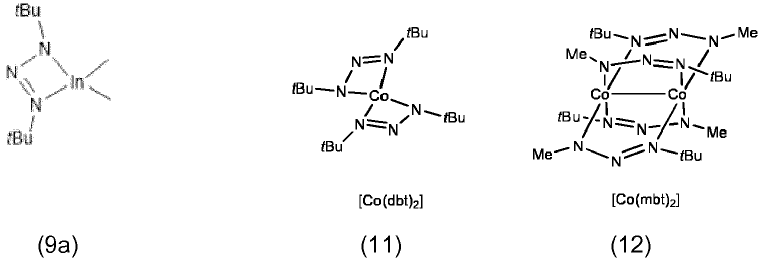

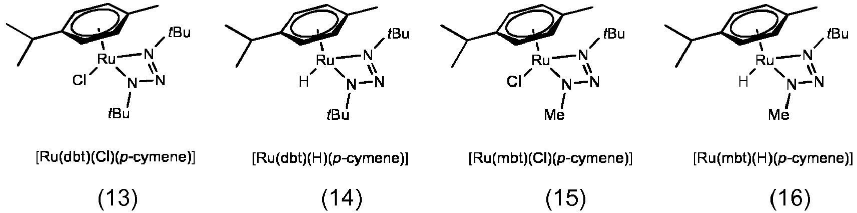

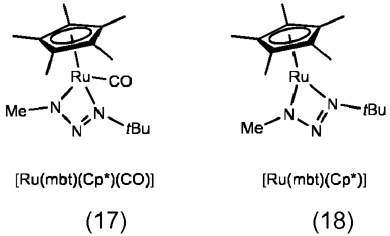

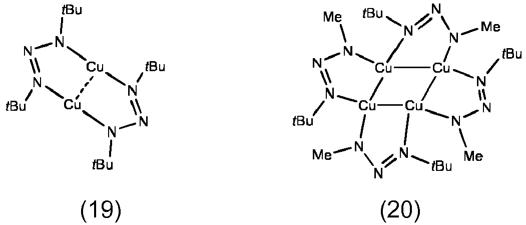

Gegenstand der Erfindung sind auch Verbindungen, die ausgewählt sind aus tert-Butyl-(N₃)H-CH₃ und Verbindungen der Formel R¹-(N₃)A-R², wobei R¹ und R² Alkylreste sind und A ein Alkalimetall ist, insbesondere Li, Na oder K, besonders bevorzugt Li.

In einer bevorzugten Ausführungsform weist die Metallverbindung eine der Formeln (21) bis (23) auf:

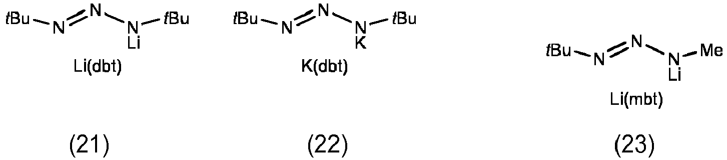

Die Reste R¹ und R² sind dabei ausgewählt wie weiter oben beschrieben. Die Alkalimetallverbindungen sind wichtige Zwischenprodukte bei der Herstellung der Metallkomplexe. Sie können auf einfache Weise wie oben beschrieben hergestellt werden und sind auf einfache Weise aus dem Reaktionsgemisch abtrennbar. Die Verbindungen sind stabil und können durch Zugabe von Wasser in die entsprechenden Neutralverbindungen (mit A=H) überführt werden. Sie eignen sich dadurch grundsätzlich auch für andere chemische Reaktionen und die Herstellung von Komplexen für andere Anwendungen als die Abscheidung von Metallen aus der Gasphase.

Die Verwendungen, Verfahren und Verbindungen lösen die oben beschriebene Aufgabe. Es werden neue und verbesserte Verbindungen bereitgestellt, um Metalle aus der Gasphase abzuscheiden. Die Verbindungen weisen eine relativ hohe Stabilität auf. Insbesondere werden Verbindungen bereitgestellt, die einen hohen Dampfdruck und gleichzeitig einen niedrigen Zersetzungspunkt aufweisen. Dadurch können die Verbindungen in die Gasphase überführt werden, ohne dass eine wesentliche Zersetzung stattfindet. Die Verbindungen weisen insbesondere bei Temperaturen, bei denen Abscheidungsverfahren aus der Gasphase üblicherweise durchgeführt werden, und dabei insbesondere von 100°C bis 300°C, eine hohe Stabilität und Volatilität auf. Die Verbindungen ermöglichen es, verschiedene Metalle aus der Gasphase abzuscheiden, wobei die metallhaltigen Beschichtungen hochrein sind. Die Erfindung betrifft zudem einfache und effiziente Verfahren zur Herstellung solcher Verbindungen. Die eingesetzten Reagenzien sind leicht zugänglich und sicher handhabbar. Die Verfahren sind mit hohen Ausbeuten durchführbar. Sie führen in wenigen Stufen, oder sogar als Eintopfverfahren, zu den gewünschten Produkten und sind bei sanften Reaktionsbedingungen durchführbar.

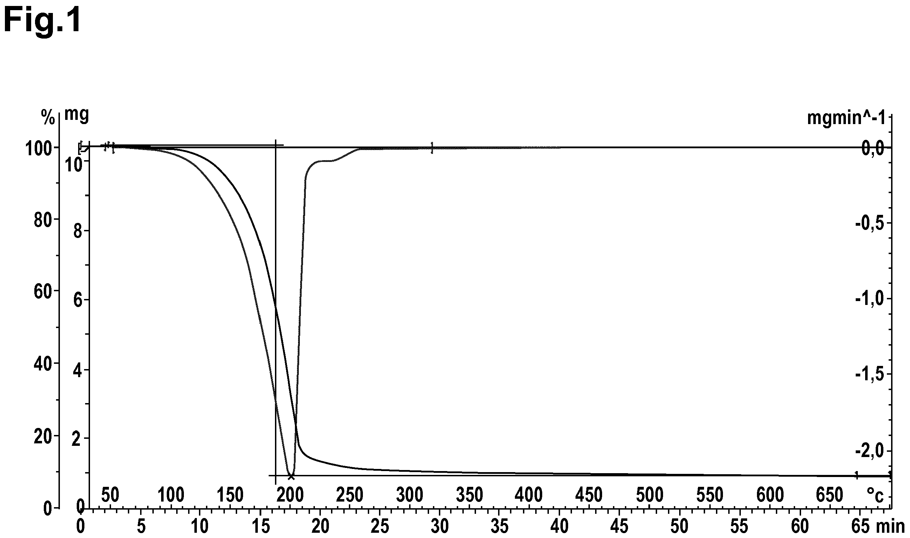

Die Figuren 1 bis 13 zeigen thermogravimetrische Analysen von Metallkomplexen (TGA), die gemäß den Ausführungsbeispielen 6, 7, 9 bis 15 und 17 bis 20 hergestellt wurden. Die TGA-Kurve zeigt, wie sich die eingesetzte Menge des Metallkomplexes mit zunehmender Temperatur verringert (in % und mg pro min und °C). Gezeigt ist jeweils auch das erste Integral der Kurve. Außerdem sind die Plateaus der Ausgangsmenge (100%) und der verbleibende Menge dargestellt, die jeweils durch eine Senkrechte bei 50% Gewichtsabnahme verbunden sind.

### Ausführungsbeispiele

### Übersicht der hergestellten Verbindungen:

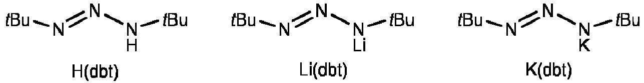

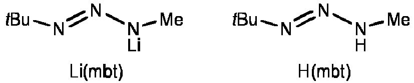

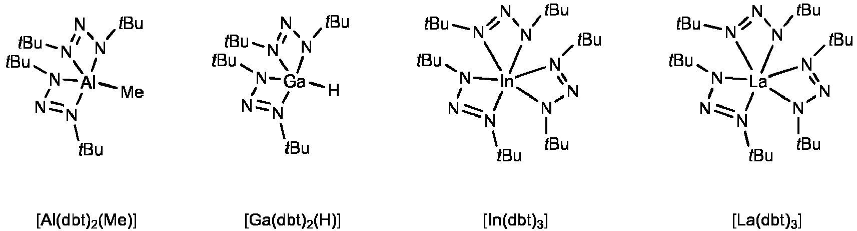

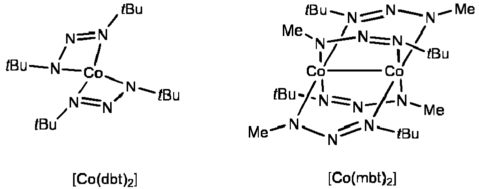

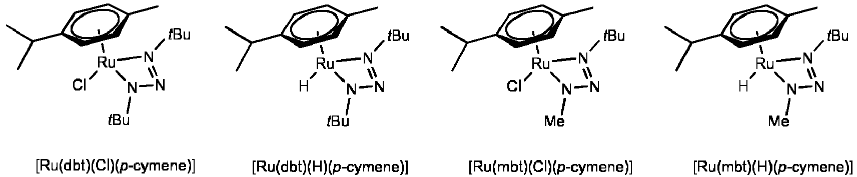

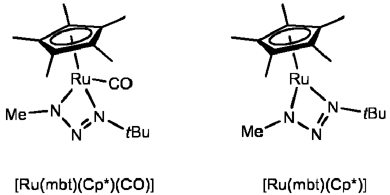

### Beispiel 1: tert-Butylazid

Im Allgemeinen werden organische Azide anhand zweier Kriterien als explosionsgefährlich eingestuft, wobei es sich bei beiden Bedingungen um empirische Grenzen der Explosivität handelt.

### 1) [(Anzahl (N-Atome) + Anzahl (O-Atome))/Anzahl (C-Atome)] < 3

### 2) Gewichtsanteil an azidischem Stickstoff > 25 ω%

Nach beiden Kriterien ist das hier beschriebene *tert*Butylazid als explosionsgefährlicher Stoff einzustufen. Unfälle bei der Darstellung oder Handhabung sind jedoch nicht bekannt. Zudem handelt es sich um eine Flüssigkeit, welche zur Aufreinigung bei 79 °C destilliert werden kann und eine N₂-Abspaltungstemperatur von circa 550 °C besitzt, welche für organische Azide vergleichsweise hoch ist.

Es gibt verschiedene synthetische Zugänge, von denen bereits seit Mitte der 1960er Jahre berichtet wurde. Eine Route geht beispielsweise von *tert*Butylchlorid aus, welches mit NaN₃ und ZnCl₂ als Katalysator in CS₂ umgesetzt wird.^{[34]} Außerdem wird in einem weiteren synthetischen Zugang *tert*Butylnitrat mit LiN₃ in DMF umgesetzt.^{[35,36]} Beide Routen können zu verschiedenen Komplikationen durch unvollständige Reaktionen oder unvollständiges Abtrennen der Nebenprodukte führen. Des Weiteren sollte der Einsatz von Kohlenstoffdisulfid aufgrund der hohen Toxizität vermieden werden. Deswegen wurde eine Route ausgehend von *tert*Butanol untersucht, bei welcher der Alkohol mit NaN₃ in einem Gemisch aus Wasser und Schwefelsäure umgesetzt wird.^{[37]} Ein Upscaling der Reaktion zum *tert*Butylazid bis zu mehreren 100 g ist laut Literatur problemlos und sicherheitstechnisch möglich. Dennoch sollte bei dieser Syntheseroute stets bedacht werden, dass im Reaktionsgemisch HN₃ gebildet wird, welches unbedingt großzügig verdünnt gehandhabt werden sollte.

### 1.1 Synthese von tertbutylazid

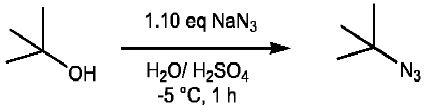

75 mL H₂O und 50 mL konzentrierte H₂SO₄ wurden bei -5 °C vorgelegt. Mithilfe eines Feststoffdosierers wurde NaN₃ (9.80 g, 151 mmol, 1.10 eq) langsam zugegeben. Die farblose Suspension wurde für 15 min gerührt und anschließend auf 0 °C erwärmt. *t*BuOH (10.2 g, 137 mmol, 1.00 eq) wurde über einen Tropftrichter langsam zugetropft. Dabei löste sich der im Reaktionsgemisch vorliegende Feststoff langsam auf. Die Reaktionslösung wurde für 16 h bei RT gerührt und anschließend in einen Scheidetrichter überführt um eine Trennung der Phasen zu erreichen. Die wässrige Phase wurde abgetrennt und umgehend mit NaOH (2 m) neutralisiert. Die organische Phase wurde zweimal mit je 20 mL NaOH (2 m) gewaschen, anschließend über Na₂SO₄ getrocknet. Das gewünschte Produkt wurde umkondensiert und konnte mit einer Ausbeute von 69% (9.40 g, 94.8 mmol) in Form einer farblosen Flüssigkeit erhalten werden.

### Beispiel 2: Synthese von H(dbt) und Li(dbt)

### 2.1 Synthese von Li(dbt) - Methode 1

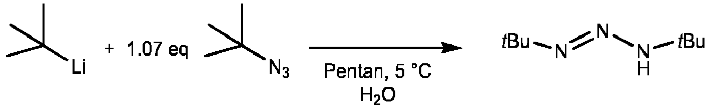

*t*BuN₃ (10.17 g, 102.6 mmol, 1.00 eq) wurde in 100 mL Pentan vorgelegt und auf 5 °C gekühlt. Innerhalb einer Stunde wurde eine Lösung von *t*BuLi in Pentan (60 mL, 1.83 m, 110 mmol, 1.07 eq) zugetropft. Der Tropftrichter wurde zweimal mit 10 mL Pentan gespült. Das Reaktionsgemisch wurde langsam erwärmt und für 1 h bei RT gerührt. Zu der leicht gelben Suspension wurden 180 mL H₂O zugeben, wodurch sich das Gemisch entfärbte. Die wässrige Phase wurde abgetrennt und die organische ein weiteres Mal mit 180 mL H₂O gewaschen. Die organische Phase wurde über Na₂SO₄ getrocknet, filtriert und anschließend wurden alle flüchtigen Bestandteile im FV entfernt. Das Rohprodukt wurde destillativ bei 60 mbar und 70 °C aufgereinigt. Die Ausbeute des gewünschten Produktes in Form einer farblosen, klaren Flüssigkeit betrug 32% (5.15 g, 32.7 mmol).

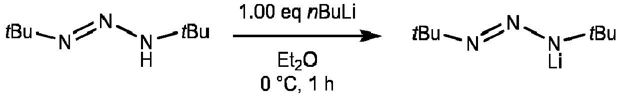

H(dbt) (700 mg, 4.45 mmol, 1.00 eq) wurde in 10 mL Et₂O vorgelegt und auf 0 °C gekühlt. Eine Lösung von *n*BuLi in Hexan (1.8 mL, 2.5 m, 4.45 mmol, 1.00 eq) wurde langsam zugetropft. Die leicht gelbe Lösung wurde für 1 h bei RT gerührt, anschließend auf RT gebracht und für weitere 24 h gerührt. Das Lösungsmittel der klaren Lösung wurde im FV entfernt, wodurch ein leicht gelber Feststoff erhalten wurde. Das gewünschte Produkt wurde im FV getrocknet und konnte mit einer Ausbeute von 95% (687 mg, 4.21 mmol) isoliert werden.

### 2.2 Synthese von Li(dbt) - Methode 2 (optimierte Synthese)

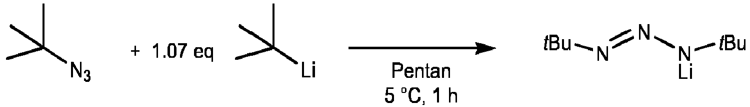

*t*BuN₃ (1.27 g, 12.8 mmol, 1.00 eq) wurde in 10 mL Pentan vorgelegt und auf 5 °C gekühlt. Eine Lösung von *t*BuLi in Hexan (7.4 mL, 1.83 m, 13.7 mmol, 1.07 eq) wurde langsam zugetropft, wobei eine leichte Gelbfärbung des Reaktionsgemisches zu beobachten war. Das Reaktionsgemisch wurde 1 h bei 5 °C gerührt, anschließend langsam auf RT erwärmt und für 1 h gerührt. Die leicht trübe Lösung wurde über einen Spritzenfilter filtriert und das Lösungsmittel des leicht gelben Filtrats wurde im FV entfernt. Das gewünschte Produkt konnte mit einer Ausbeute von 83% (1.64 g, 10.0 mmol) erhalten werden.

Anhand durchgeführter analytischer Untersuchungen konnten keine Unterschiede zwischen den beiden Chargen Li(dbt) beobachtet werden. Bei der optimierten Synthese des Li(dbt) handelt es sich um eine einstufige Synthese ohne Nebenprodukte. Während zunächst vom Neutralligand ausgegangen wurde, führt die Optimierung sowohl zum Einsparen an Reagenzien als auch verschiedenster Arbeitsschritte, u.a. einer Destillation.

### Beispiel 3: Synthese von K(dbt)

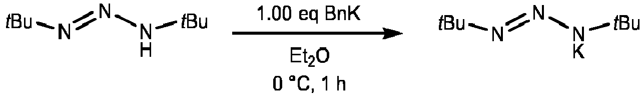

BnK (700 mg, 4.45 mmol, 1.00 eq) wurde in 10 mL Et₂O aufgenommen und bei 0 °C tropfenweise mit H(dbt) (582 mg, 4.47 mmol, 1.00 eq) versetzt. Die farblose Suspension wurde auf RT erwärmt und für 16 h gerührt. Die leicht trübe Lösung wurde filtriert, das Lösungsmittel des farblosen Filtrats wurde im Unterdruck vollständig entfernt. Es konnten 85% (510 mg, 2.61 mmol) des gewünschten Produktes in Form eines farblosen Feststoffs erhalten werden.

### Beispiel 4: Synthese von Li(mbt)

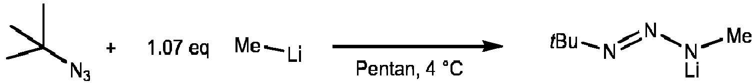

*t*BuN₃ (1.00 g, 10.7 mmol, 1.00 eq) wurde in 15 mL Pentan vorgelegt und auf 4 °C gekühlt. Eine Lösung von MeLi in Et₂O (6.8 mL, 1.60 m, 10.7 mmol, 1.07 eq) wurde langsam tropfenweise zur Reaktionslösung zugegeben, wodurch ein farbloser Feststoff ausfiel. Das Reaktionsgemisch wurde auf RT erwärmt, der entstandene farblose Niederschlag abfiltriert und im FV für mehrere Stunden getrocknet. Das gewünschte Produkt konnte als farbloser Feststoff mit einer Ausbeute von 87% (1.13 g, 9.31 mmol) erhalten werden. Ein Upscaling der Reaktion ist möglich.

### Beispiel 5: Synthese von H(mbt)

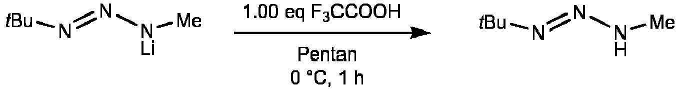

Li(mbt) (143 mg, 1.18 mmol, 1.00 eq) wurde in Pentan vorgelegt und auf 0 °C gekühlt. Unter Rühren wurde F₃CCOOH (135 mg, 1.18 mmol, 1.00 eq) langsam zugetropft, wobei ein leichtes Aufschäumen der Reaktionslösung beobachtet werden konnte. Das eingesetzte Li(mbt) ging beim Erwärmen des Reaktionsgemischs auf RT in Lösung und die leicht trübe Suspension wurde für 16 h bei RT gerührt. Nachdem die Suspension über einen Spritzenfilter filtriert und das Pentan im FV entfernt wurde, konnte das gewünschte Produkt in Form einer farblosen Flüssigkeit erhalten werden.

### Beispiel 6: Synthese von [Al(dbt)₂(Me)]

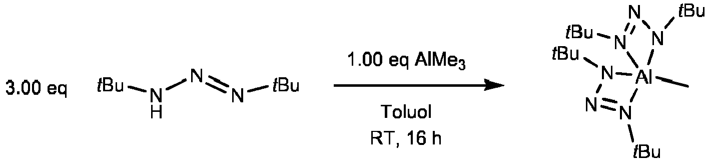

Eine toluolische Lösung von AlMe₃ (33 mg, 0.46 mmol, 1.00 eq) wurde vorgelegt und bei RT tropfenweise mit H(dbt) (300 mg, 1.91 mmol, 3.00 eq) versetzt. Dabei konnte umgehend eine Gasentwicklung beobachtet werden. Das farblose Reaktionsgemisch wurde für 72 h bei RT gerührt und anschließend über einen Spritzenfilter filtriert. Das Lösungsmittel des Filtrats wurde im FV entfernt, wodurch ein farbloses Öl erhalten wurde. Nach mehrmaligem Gefriertrocknen wurde das gewünschte Produkt mit einer Ausbeute von 87% (276 mg, 0.56 mmol) als farbloser Feststoff erhalten (Schmelzpunkt: 46 °C).

### Thermogravimetrische Untersuchung von [Al(dbt)₂(Me)]

Das Rohprodukt wurde mittels thermogravimetrischer Analyse bis 700°C bei 10 K/min untersucht (FIG. 1). Die thermogravimetrische Analyse zeigt einen einstufigen Prozess mit einem Gesamtmasseabbau von circa 91,2%. Eine Gewichtsabnahme von 3% der Ausgangssubstanz wurde bei 127,2°C festgestellt.

### Beispiel 7: Synthese von [Ga(dbt)₂(H)]

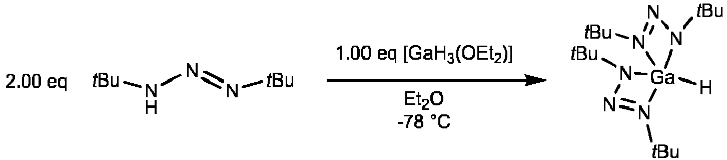

Eine auf -78 °C gekühlte Lösung von GaCl₃ (440 mg, 2.50 mmol, 1.00 eq) in 5 mL Et₂O wurde zu einer Suspension aus LiH (260 mg, 32.8 mmol, 13.1 eq) in 5 mL Et₂O getropft. Dabei fiel sofort ein leicht grauer Niederschlag aus. Das Reaktionsgemisch wurde für 2 h bei -78 °C, anschließend für 16 h bei RT gerührt. Die leicht graue Suspension wurde in einen auf vorgekühlten Kolben filtriert. Zu dem klaren Filtrat wurde bei -78 °C eine auf -78 °C gekühlte Lösung aus GaCl₃ (176 mg, 1.00 mmol, 0.40 eq) in 5 mL Et₂O getropft. Die Suspension wurde unter Rühren bis auf 0 °C erwärmt und anschließend in einen auf -78 °C vorgekühlten Kolben filtriert. Das klare Filtrat wurde bei 0 °C zu einer Lösung aus H(dbt) (786 mg, 5.00 mmol, 2.00 eq) in 5 mL Et₂O zugetropft. Dabei war umgehend eine Gasentwicklung zu beobachten. Die Suspension wurde langsam auf RT erwärmt und für 16 h gerührt. Das Gemisch wurde über einen Spritzenfilter filtriert und alle flüchtigen Bestandteile wurden im FV entfernt. Es blieb ein farbloser Feststoff zurück, welcher in 5 mL Hexan aufgenommen und erneut über einen Spritzenfilter filtriert wurde. Das Lösungsmittel des Filtrats wurde im FV entfernt, wodurch das Produkt mit einer Ausbeute 53% (508 mg, 1.33 mmol, Schmelzpunkt: 46 °C) erhalten wurde. Einkristalle für die Strukturanalyse konnten durch Sublimation im FV bei 60 °C erhalten werden.

Der entsprechende Dihydrido-Gallium-Komplex [Ga(dbt)H₂] konnte nicht durch die 1:1-Umsetzung von H(dbt) und [GaH₃(OEt₂)] erhalten werden.

### Thermogravimetrische Untersuchung von [Ga(dbt)₂(H)]

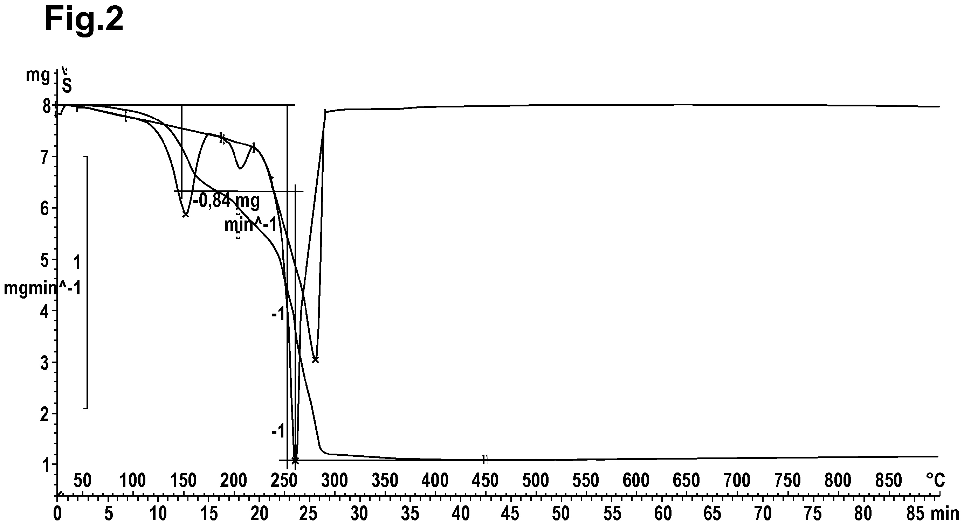

Das Rohprodukt wurde mittels thermogravimetrischer Analyse bis 900°C bei 10 K/min untersucht (FIG. 2). Insgesamt wurden 86,6% der Ausgangssubstanz in die Gasphase überführt.

### Beispiel 8: Synthese von [In(dbt)Me₂]

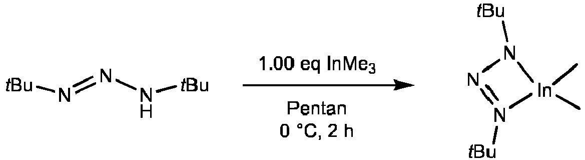

H(dbt) (250 mg, 1.59 mmol, 1.00 eq) wurde in 8 mL Pentan vorgelegt und auf 0 °C gekühlt. Eine toluolische Lösung von InMe₃ (254 mg, 1.59 mmol, 1.00 eq) wurde tropfenweise zugegeben, wobei eine leichte Gasentwicklung zu beobachten war. Das Reaktionsgemisch wurde für 1 h bei 0 °C und für 16 h bei RT gerührt. Das Lösungsmittel der klaren Lösung wurde im FV entfernt, wodurch das gewünschte Produkt als farblose Flüssigkeit isoliert werden konnte. Zur Aufreinigung kann das Produkt bei 40 °C im leichten Vakuum umkondensiert werden.

Die Reaktion kann in analoger Weise auch in Toluol durchgeführt werden, jedoch führt dies aufgrund der hohen Flüchtigkeit des Produktes zu Schwierigkeiten bei der Isolierung des Indium-Komplexes.

### Beispiel 9: Synthese von [In(dbt)₃]

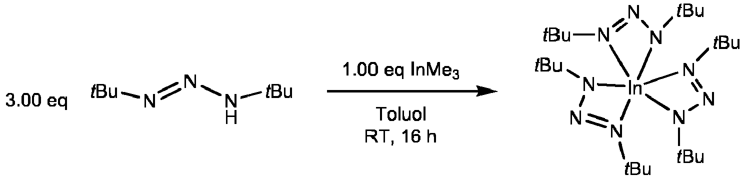

InMe₃ (150 mg, 0.94 mmol, 1.00 eq) wurde in 5 mL Toluol vorgelegt, auf 0 °C gekühlt und tropfenweise mit H(dbt) (444 mg, 2.83 mmol, 3.00 eq) versetzt. Das Reaktionsgemisch wurde langsam auf RT erwärmt und für 16 h gerührt. Das Lösungsmittel der klaren Lösung wurde im FV entfernt. Das gewünschte Produkt wurde in Form eines farblosen Feststoffs mit einer Ausbeute von 73% (403 mg, 0.69 mmol) erhalten und kann im FV bei 80 °C sublimiert werden.

### Thermogravimetrische Untersuchung von [In(dbt)₃]

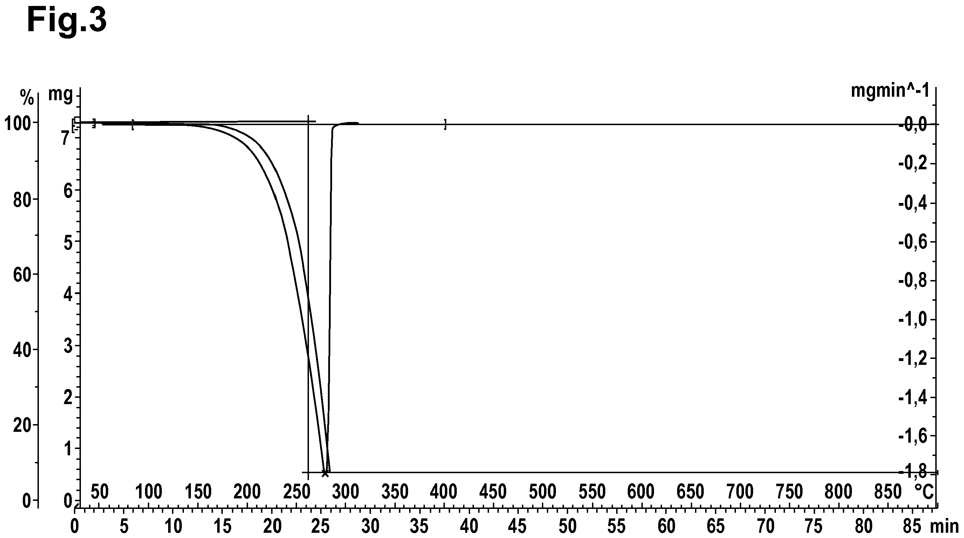

Das Rohprodukt wurde mittels thermogravimetrischer Analyse bis 900°C bei 10 K/min untersucht (FIG. 3). Die Analyse zeigt einen einstufigen Prozess mit einem Gesamtmasseabbau von circa 92,8%. Eine Gewichtsabnahme von 3% der Ausgangssubstanz wurde bei 196,7°C festgestellt.

### Beispiel 10: Synthese von [La(dbt)₃]

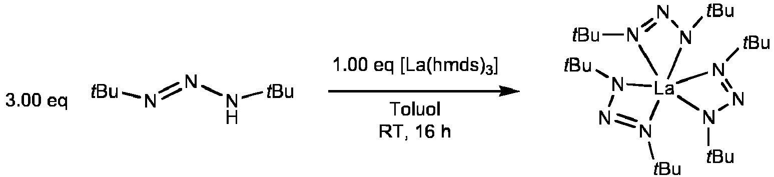

[La(hmds)₃] (225 mg, 0.36 mmol, 1.00 eq) wurde in 10 mL Toluol vorgelegt und auf 0 °C gekühlt. Tropfenweise wurde H(dbt) (171 mg, 1.09 mmol, 3.00 eq) zu der farblosen Lösung gegeben. Das klare, farblose Reaktionsgemisch wurde auf RT erwärmt und für 16 h gerührt. Mithilfe einer ¹H-NMR-Reaktionskontrolle wurde bestätigt, dass kein [La(hmds)₃] mehr im Reaktionsgemisch vorhanden war. Alle flüchtigen Bestandteile der Reaktionslösung wurden im FV entfernt und der erhaltene farblose Feststoff wurde bei 60 °C im FV getrocknet. Das gewünschte Produkt wurde mit einer Ausbeute von 72% (158 mg, 0.26 mmol) erhalten.

### Thermogravimetrische Untersuchung von [La(dbt)₃]

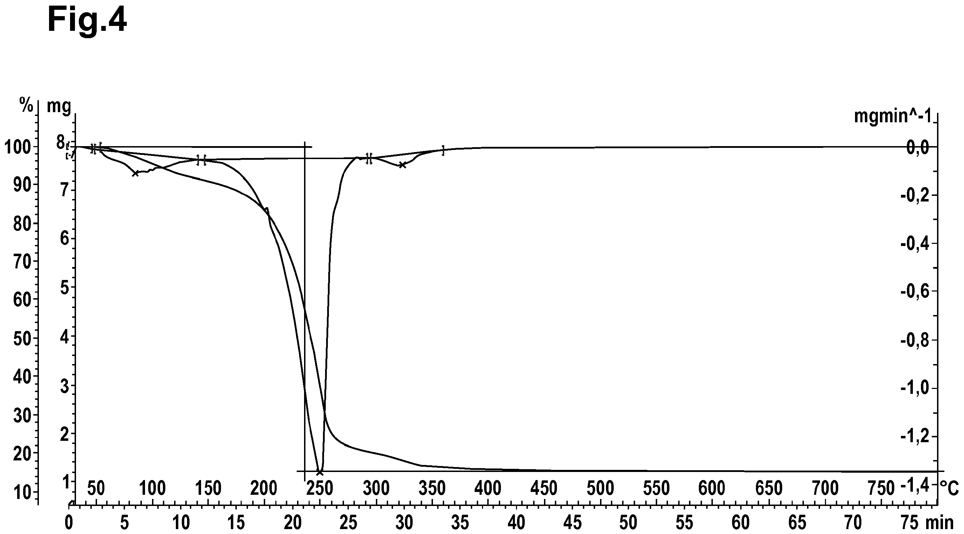

Das Rohprodukt wurde mittels thermogravimetrischer Analyse bis 800°C bei 10 K/min untersucht (FIG. 4). Die thermogravimetrische Analyse zeigt einen einstufigen Prozess mit einem Gesamtmasseabbau von circa 85%. Eine Gewichtsabnahme von 3% der Ausgangssubstanz wurde bei 85°C festgestellt. Im Bereich bis 100 °C lässt sich erkennen, dass noch Restspuren Hhmds im Produkt vorhanden sind. Ab einer Temperatur von 103 °C kommt es zu einem einsetzenden Schmelzprozess des Lanthan-Komplexes, wobei der maximale Masseabbau bei einer Temperatur von 250 °C erreicht ist. Ab einer Temperatur von ungefähr 400 °C lässt sich kein signifikanter Masseabbau mehr beobachten.

### Beispiel 11: Synthese von [Co(dbt)₂]

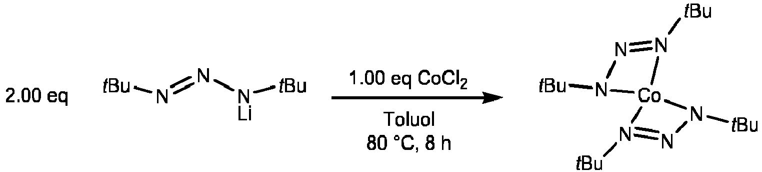

Li(dbt) (654 mg, 4.00 mmol, 2.00 eq) wurde zusammen mit CoCl₂ (260 mg, 2.00 mmol, 1.00 eq) vorgelegt und mit 15 mL Toluol versetzt. Das Reaktionsgemisch wurde für 8 h auf 80 °C erhitzt, wobei ein Farbwechsel von blau zu dunkelrot beobachtet werden konnte. Das Lösungsmittel des abgekühlten Reaktionsgemisches wurde im FV entfernt und das gewünschte Produkt wurde direkt aus dem Rückstand im FV bei 100 °C sublimiert. [Co(dbt)₂] wurde in Form eines dunkelroten, fast schwarzen Feststoffs mit einer Ausbeute von 53% (394 mg, 1.06 mmol) erhalten.

### Thermogravimetrische Analyse und Rückstandsbestimmung von [Co(dbt)₂]

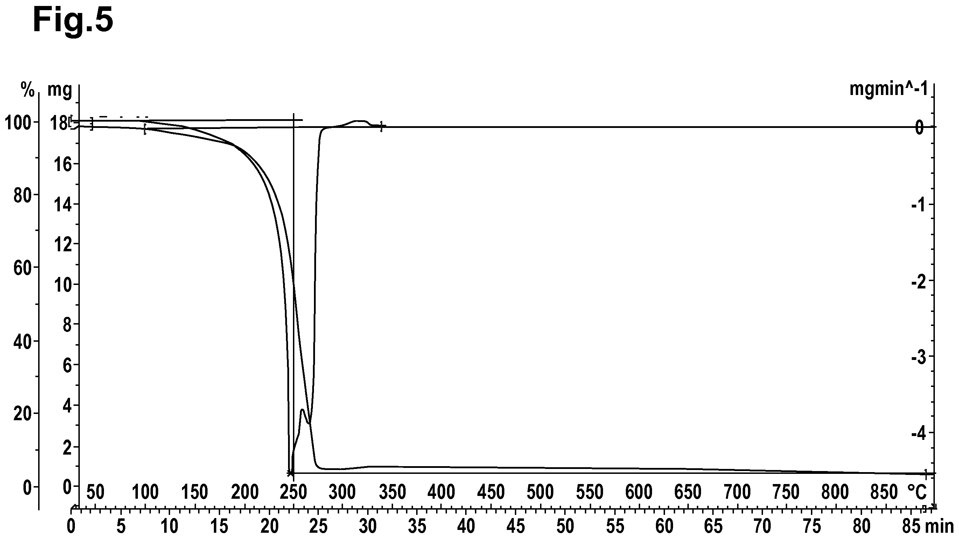

In der TGA-Kurve bis 900°C bei 10 K/min (FIG. 5) lässt sich ein einstufiger Masseverlust beobachten, bei dem der maximale Masseabbau pro Zeit bei einer Temperatur von 248 °C beobachtet werden kann. Bei einer Temperatur von 156 °C hat sich die Probe zu 3% abgebaut. Ab einer Temperatur von 91 °C lässt sich ein endothermer, sehr breiter Peak beobachten, welcher einem unregelmäßigen Schmelzvorgang zugeordnet werden kann. Der Gesamtmasseverlust liegt bei 97%, wobei der erhaltene Rückstand mittels XRPD näher untersucht wurde. Dabei zeigte sich, dass es sich bei dem Rückstand um elementares Cobalt handelt.

### Beispiel 12: Synthese von [Co₂(mbt)₄]

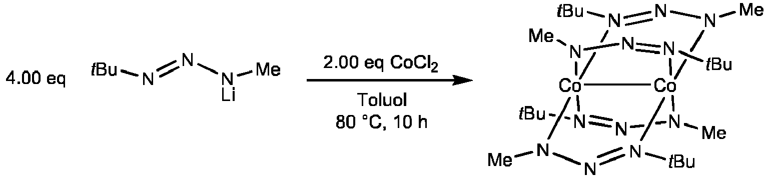

Li(mbt) (193 mg, 1.59 mmol, 4.00 eq) und CoCl₂ (103 mg, 0.79 mmol, 2.00 eq) wurden zusammen vorgelegt und bei 0 °C mit 10 mL Toluol versetzt. Beim Auftauen des Reaktionsgemisches konnte ein Farbwechsel von blau zu dunkelbraun beobachtet werden. Das Reaktionsgemisch wurde für 10 h auf 80 °C erhitzt und anschließend wurde das Lösungsmittel im FV bei RT entfernt. Aus dem dunkelbraunen Rückstand wurde das gewünschte Produkt im dynamischen Vakuum bei 85 °C sublimiert. Der dinukleare Cobalt-Komplex konnte als rotbrauner Feststoff mit einer Ausbeute von 51% (116 mg, 0.20 mmol) erhalten werden. Einkristalle für die Kristallstrukturanalyse konnten durch Sublimation im leichten Vakuumbei 100 °C erhalten werden. Aufgrund von Spinpaarung der beiden Cobalt-Kerne im [Co₂(mbt)₄], zeigt dieses im Gegensatz zu [Co(dbt)₂] diamagnetisches Verhalten.

### Thermogravimetrische Analyse und Rückstandsbestimmung von [Co₂(mbt)₄]

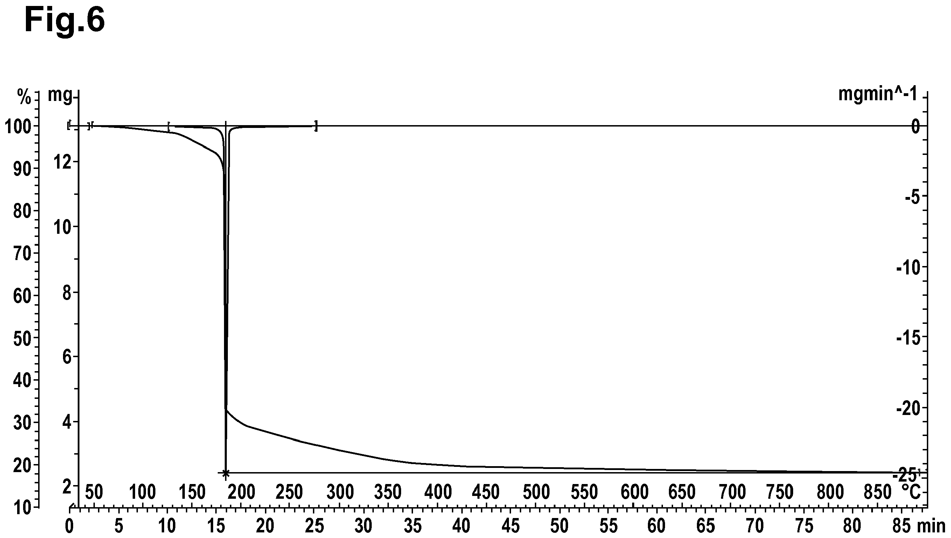

Die TGA-Kurve bis 900°C bei 10 K/min (FIG. 6) des Cobalt-Komplexes zeigt einen einstufigen Verlauf, wobei der maximale Masseabbau pro Zeit bei einer Temperatur von 189 °C beobachtet werden kann. Der 3%- Abbau wurde bei einer Temperatur von 147 °C ermittelt. Ab einer Temperatur von 184 °C lässt sich ein einsetzender exothermer Prozess erkennen, welcher zum maximalen Masseabbau pro Zeit führt und einem Zersetzungsprozess zugeordnet werden kann. Der Gesamtmasseabbau liegt bei 82%, wobei der aus dieser Messung erhaltene Rückstand mittels XRPD untersucht wurde. Dieser konnte als elementares Cobalt identifiziert werden.

### Beispiel 13: Synthese von [Ru(dbt)(Cl)(p-cymene)]

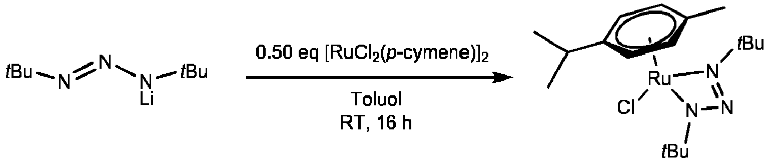

Li(dbt) (248 mg, 1.52 mmol, 1.00 eq) wurde in 5 mL Toluol vorgelegt. Der Ruthenium-Präkursor (465 mg, 0.76 mmol, 0.50 eq) wurde portionsweise zugegeben und es wurde mit 4 mL Toluol nachgespült. Nach kurzer Zeit war ein Farbwechsel von dunkelrot nach schwarz zu beobachten. Das Reaktionsgemisch wurde über Nacht bei RT gerührt und anschließend über einen Spritzenfilter filtriert. Das Lösungsmittel des gelb-schwarzen Filtrats wurde im FV entfernt. Das gewünschte Produkt konnte in Form eines dunkelgrünen Feststoffs mit einer Ausbeute von 51% (331 mg, 0.77 mmol, Schmelzpunkt: 66.5 °C).

### Thermogravimetrische Untersuchung von [Ru(dbt)(Cl)(p-cymene)]

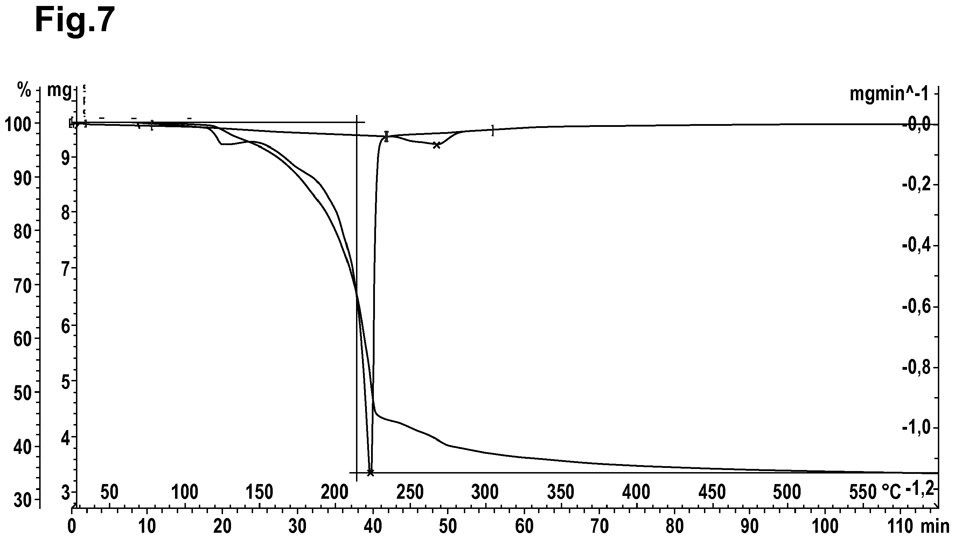

Die TGA-Kurve bis 600°C bei 5 K/min (FIG. 7) zeigt einen einstufigen Verlauf mit einem maximalen Masseverlust pro Zeit bei 235 °C. Während der 3%-Abbau bei einer Temperatur von 153 °C liegt, beträgt der Gesamtmasseabbau 67%. Bei einer Temperatur von 67 °C setzt ein Schmelzvorgang ein, welcher fast übergangslos in einen Sublimationsvorgang übergeht. Ab einer Temperatur von 200 °C lässt sich anhand der SDTA-Kurve ein weiterer exothermer Peak beobachten, welcher dem Zersetzungsvorgang zugeordnet werden kann (nicht gezeigt). Ab einer Temperatur von circa 400 °C verändert sich der Masseabbau nicht mehr signifikant. Der bei der Messung erhaltene Rückstand wurde mittels XRPD näher untersucht und elementares Ruthenium konnte nachgewiesen werden.

### Beispiel 14: Synthese von [Ru(dbt)(H)(p-cymene)]

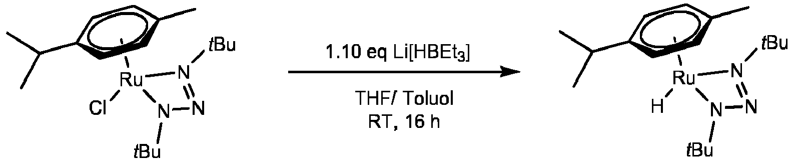

[Ru(dbt)(Cl)(*p*-cymene)] (227 mg, 0.51 mmol, 1.00 eq) wurde in 5 mL Toluol vorgelegt. Bei 0 °C wurde eine Lösung von Li[HBEt₃] in THF (0.56 mL, 1 m, 0.56 mmol, 1.10 eq) vorgelegt. Das Reaktionsgemisch wurde langsam auf RT erwärmt und für 16 h gerührt. Dabei konnte das Ausfallen eines farblosen Feststoffs beobachtet werden. Durch Filtration wurde der ausgefallene Feststoff abgetrennt und das Lösungsmittel des Filtrats wurde im FV entfernt. Das gewünschte Produkt konnte nach mehrmaligem Gefriertrocknen in Form einer grün-schwarzen viskosen Flüssigkeit erhalten werden. Zur Aufreinigung wurde der Ruthenium-Hydrido-Komplex im FV bei 95 °C umkondensiert.

Die Synthese kann auch alternativ mit LiAlH₄ (0.25 Äquivalente) durchgeführt werden. Der methyl-substituierte Komplex, welcher durch die Umsetzung von [Ru(dbt)(CI)(p-cymene)] und Methyllithium dargestellt werden kann, liegt hingegen als Feststoff vor.

### Thermogravimetrische Untersuchungen von [Ru(dbt)(H)(p-cymene)]

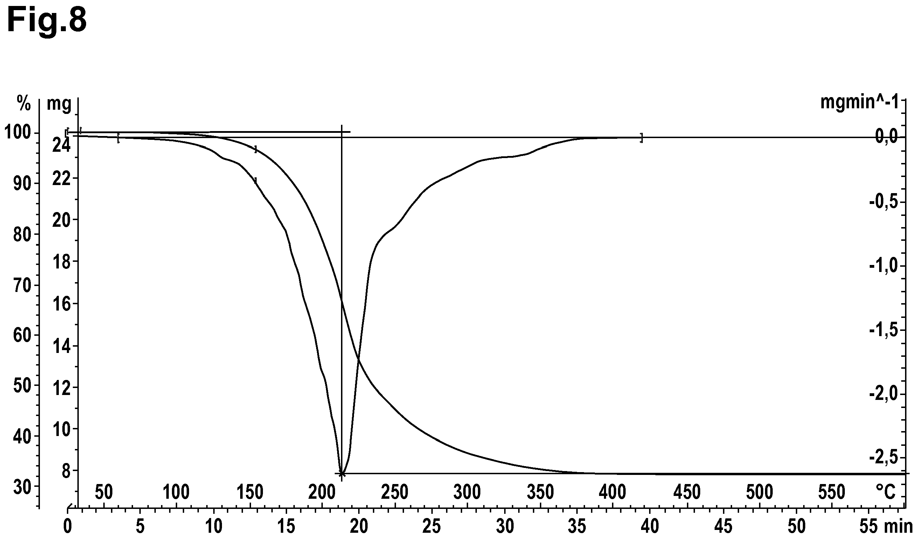

Das Rohprodukt wurde mittels thermogravimetrischer Analyse bis 600°C bei 10 K/min untersucht (FIG. 8). Die Analyse zeigt einen einstufigen Prozess mit einem Gesamtmasseabbau von circa 67,8%. Eine Gewichtsabnahme von 3% der Ausgangssubstanz wurde bei 150,5°C festgestellt.

### Beispiel 15: Synthese von [Ru(mbt)(Cl)(p-cymene)]

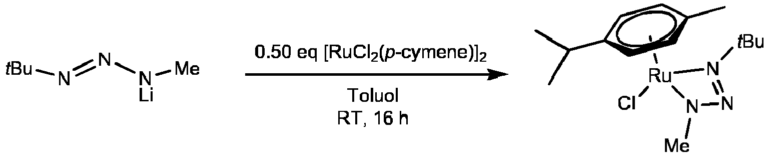

Li(mbt) (221 mg, 1.82 mmol, 1.00 eq) wurde in 10 mL Toluol vorgelegt und bei 0 °C portionsweise mit [RuCl₂(*p*-cymene)]₂ (559 mg, 0.91 mmol, 0.50 eq) versetzt. Beim langsamen Auftauen des Gemisches auf RT konnte ein Farbwechsel von braun zu dunkelgrün beobachtet werden. Das Reaktionsgemisch wurde für 16 h bei RT gerührt und anschließend über einen Spritzenfilter filtriert. Das Lösungsmittel des rötlichen Filtrats wurde im FV entfernt und der zurückgebliebene Feststoff wurde mehrfach gefriergetrocknet. Das gewünschte Produkt wurde in Form eines dunkelroten Feststoffs mit einer Ausbeute von 59% (414 mg, 1.07 mmol, Schmelzpunkt: 51.9 °C) erhalten.

### Thermogravimetrische Untersuchung von [Ru(mbt)(Cl)(p-cymene)]

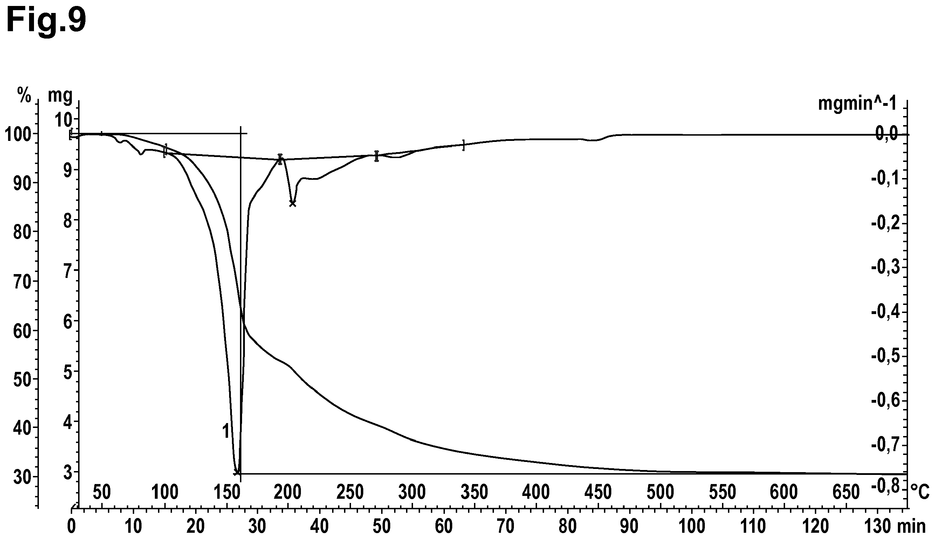

Die gemessene TGA/SDTA-Kurve bis 700°C bei 5 K/min (FIG. 9) zeigt einen einstufigen Masseabbau, mit einem maximalen Abbau bei einer Temperatur von 169 °C. Oberhalb dieser Temperatur nimmt der Masseverlust pro Zeit erneut ab, was in sich anhand einer positiven Steigung ab dem Minima beobachten lässt. Der 3%-Abbau des Ruthenium-Komplexes liegt bei 118 °C. Ab einer Temperatur von 52 °C lässt sich ein einsetzender Schmelzvorgang beobachten, welcher stufenlos in einen Sublimationsvorgang übergeht. Der Gesamtmasseverlust liegt bei 64%. Der aus dieser Messung erhaltene Rückstand wurde mittels XRPD Analyse untersucht und konnte als elementares Ruthenium identifiziert werden.

### Beispiel 16: Synthese von [Ru(mbt)(H)(p-cymene)]

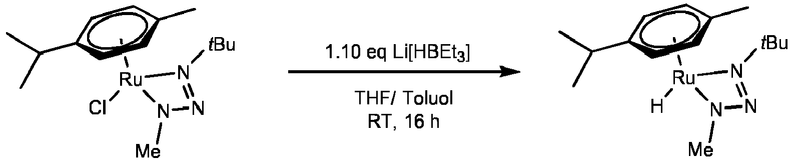

[Ru(mbt)(Cl)(*p*-cymene)] (196 mg, 0.49 mmol, 1.00 eq) wurde in 5 mL Toluol gelöst und bei 0 °C mit einer Lösung von Li[HBEt₃] in THF (0.54 mL, 1 m, 0.54 mmol, 1.10 eq) versetzt. Das Reaktionsgemisch wurde langsam auf RT erwärmt und für 16 h gerührt, wobei ein farbloser Feststoff ausfiel. Dieser wurde abgetrennt und das Lösungsmittel des Filtrats wurde im FV entfernt. Das gewünschte Produkt konnte nach mehrmaligem Gefriertrocknen in Form einer schwarzen viskosen Flüssigkeit erhalten werden.

### Beispiel 17: Synthese von [Ru(mbt)(Cp*)(CO)]

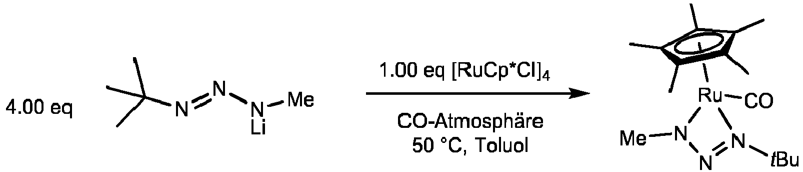

[RuCp*Cl]₄ (90 mg, 0.45 mmol, 1.00 eq) und Li(mbt) (216 mg, 1.79 mmol, 4.00 eq) wurden zusammen vorgelegt und in 25 mL Toluol aufgenommen. Die dunkelbraune Lösung wurde für 1 h auf 50 °C erhitzt, anschließend auf RT abgekühlt. Für 1 h wurde CO bei RT durch das Reaktionsgemisch geleitet. Die dunkelrot-schwarze Reaktionslösung wurde über Celite® filtriert. Das Lösungsmittel wurde im FV entfernt, wodurch ein dunkelroter, fast schwarzer, honigartiger Feststoff zurückblieb, welcher als gewünschtes Produkt identifiziert werden konnte.

### Thermogravimetrische Untersuchung des Rohprodukts [Ru(mbt)(Cp*)(CO)]

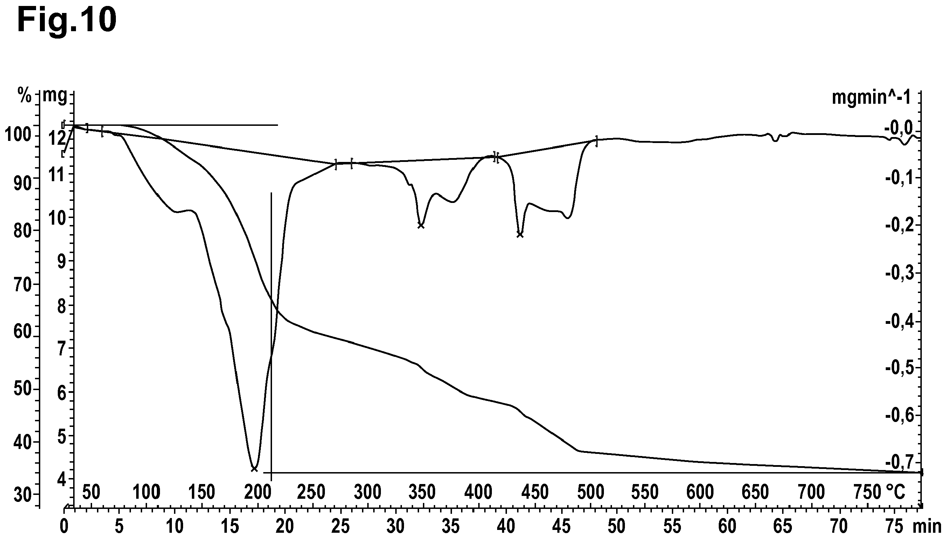

Das Rohprodukt wurde bis 800°C bei 10 K/min mittels thermogravimetrischer Analyse untersucht (FIG. 10). Dabei lässt sich ein stufenweiser Abbau beobachten, mit einem Gesamtmasseabbau von 67%. Der Abbau beginnt bei einer Temperatur von circa 75 °C mit einem endothermen Peak. Eine Gewichtsabnahme von 3% der Ausgangssubstanz wurde bei 116,0°C festgestellt. Der aus dieser Messung erhaltene Rückstand wurde mittels XRPD untersucht und konnte als elementares Ruthenium identifiziert werden.

### Beispiel 18: Synthese von [Ru(mbt)(Cp*)]

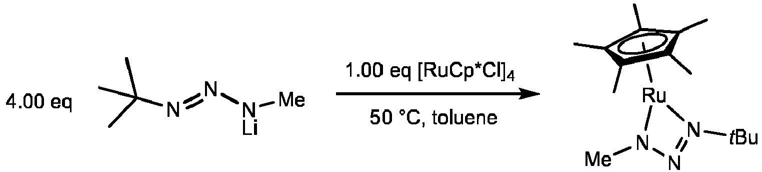

[RuCp*Cl]₄ (100 mg, 0.50 mmol, 1.00 eq) und Li(mbt) (244 mg, 1.98 mmol, 4.00 eq) wurden zusammen vorgelegt und in 10 mL Toluol aufgenommen. Die dunkelbraune Suspension wurde für 2 h auf 50 °C erhitzt und anschließend auf RT abgekühlt. Das dunkelrot-schwarze Reaktionsgemisch wurde über Celite® filtriert und das Lösungsmittel des Filtrats wurde im FV entfernt, wodurch ein dunkelgrüner, fast schwarzer, honigartiger Feststoff isoliert wurde.

### Thermogravimetrische Untersuchung des Rohprodukts [Ru(mbt)(Cp*)]

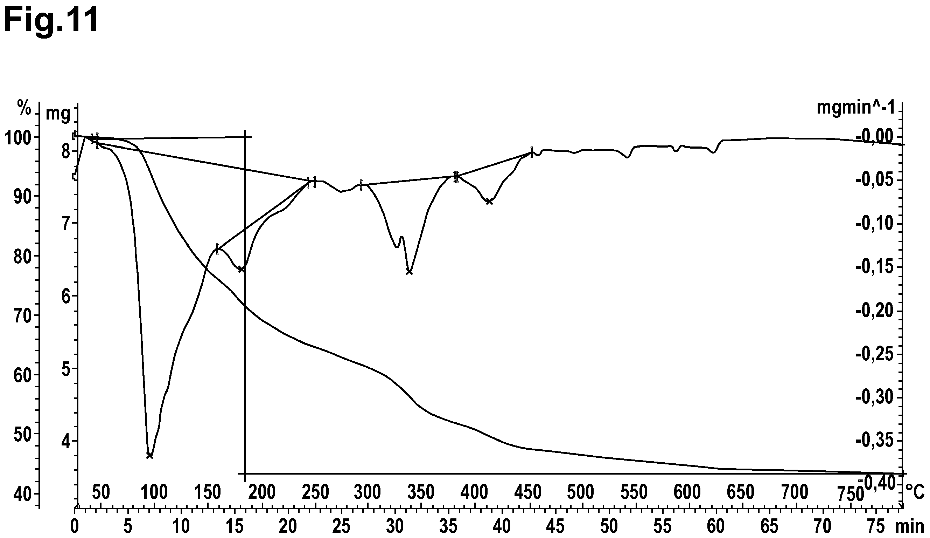

Das Rohprodukt wurde mittels thermogravimetrischer Analyse bis 800°C bei 10 K/min untersucht (FIG. 11), wobei sich ein stufenweiser Abbau beobachten lässt. Der Gesamtmasseabbau liegt bei 57%. Der Abbau beginnt bei einer Temperatur von circa 79 °C mit einem endothermen Peak. Eine Gewichtsabnahme von 3% der Ausgangssubstanz wurde bei 87,6°C festgestellt. Der aus dieser Messung erhaltene Rückstand wurde mittels XRPD untersucht und konnte als elementares Ruthenium identifiziert werden.

### Beispiel 19: Synthese von [Cu₂(dbt)₂]

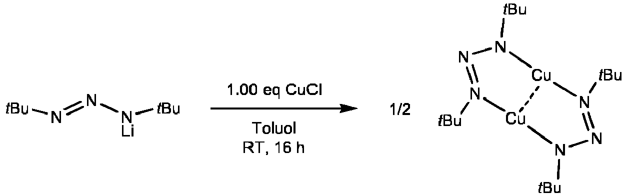

Li(dbt) (280 mg, 1.72 mmol, 1.00 eq) wurde zusammen mit CuCl (170 mg, 1.72 mmol, 1.00 eq) vorgelegt und mit 10 mL vorgekühlten Toluol versetzt. Das Reaktionsgemisch wurde für 16 h bei RT gerührt, wobei ein Farbwechsel von gelb zu braun beobachtet werden konnte. Die Suspension wurde über Celite® filtriert und anschließend das Lösungsmittel des Filtrats im FV entfernt. Das Rohprodukt wurde im dynamischen Vakuum bei 70 °C sublimativ aufgereinigt und konnte mit einer Ausbeute von 71% (268 mg, 0.61 mmol) als gelber Feststoff erhalten werden. Einkristalle für die Kristallstrukturanalyse wurden aus einer gesättigten Lösung von n-Hexan bei -21 °C erhalten.

### Thermogravimetrische Untersuchung

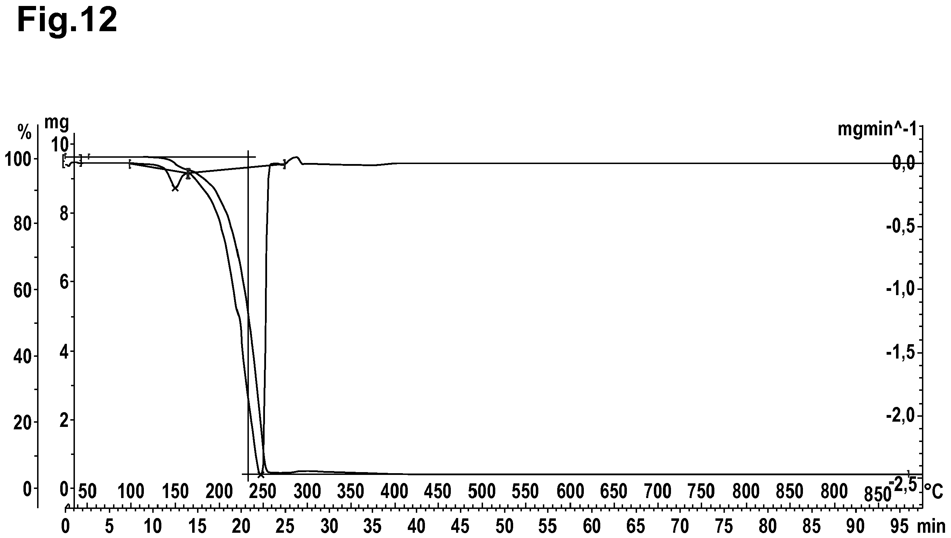

Das Rohprodukt wurde mittels thermogravimetrischer Analyse bis 1000°C bei 10 K/min untersucht (FIG. 12). [Cu₂(dbt)₂] zeigt einen einstufigen Masseabbau, wobei der 3%-Abbau bei 156 °C liegt. Der Gesamtmassenverlust liegt bei 96%, was auf die gute Sublimierbarkeit der Kupfer-Verbindung zurückzuführen ist. Ab einer Temperatur von circa 260 °C ist kein signifikanter Masseabbau mehr zu beobachten. Als Rückstand wurde elementares Kupfer im Tiegel beobachtet.

### Beispiel 20: Synthese von [Cu₄(mbt)₄]

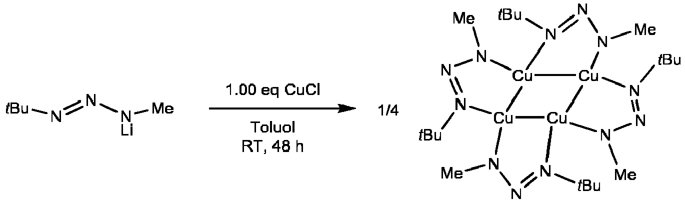

Li(mbt) (208 mg, 1.72 mmol, 1.00 eq) und CuCl(170mg, 1.72 mmol, 1.00 eq) wurden vorgelegt und mit 10 mL vorgekühlten Toluol versetzt. Das farblose Reaktionsgemisch wurde für 48 h bei RT gerührt, wodurch eine Farbänderung zu leuchtend gelb beobachtet werden konnte. Die Suspension wurde über Celite® filtriert und das Lösungsmittel des Filtrats wurde im FV entfernt. Das leuchtend gelbe Rohprodukt wurde im dynamischen Vakuum bei 65 °C bis 75 °C sublimativ aufgereinigt und konnte mit einer Ausbeute von 82% (248 mg, 0.35 mmol) erhalten werden. Einkristalle für die Kristallstrukturanalyse konnten aus einer gesättigten Lösung von n-Hexan bei RT erhalten werden.

### Thermogravimetrische Untersuchung

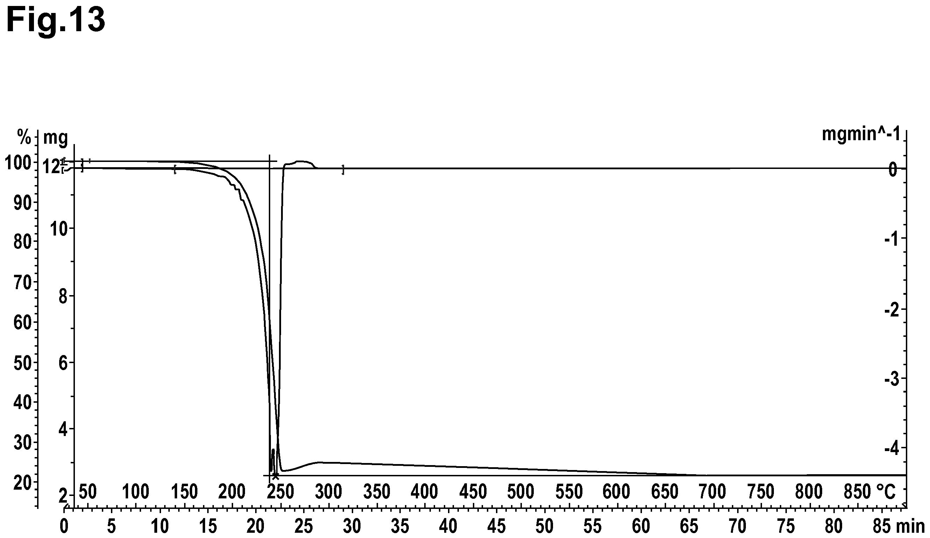

Das Rohprodukt wurde mittels thermogravimetrischer Analyse bis 900°C bei 10 K/min untersucht (FIG. 13). [Cu₄(mbt)₄] zeigt einen einstufigen Abbau mit einem Gesamtmassenverlust von 79%. Der 3%-Abbau liegt bei 198 °C, während der maximale Masseabbau bei circa 250 °C bestimmt wurde. Ab einer Temperatur von 255 °C lässt sich kein signifikanter Masseverlust mehr beobachten. Die SDTA-Kurve zeigt verschiedene Schmelz- bzw. Phasenumwandlungs-prozesse bis zur einsetzenden Zersetzung, welche bei einer Temperatur von 236 °C beginnt (nicht gezeigt). Der Rückstand aus der thermogravimetrischen Untersuchung wurde mittels XRPD untersucht und konnte als elementares Kupfer identifiziert werden.

### Literaturverzeichnis

[1] J. C. Brand, B. P. Roberts, J. Chem. Soc. Chem. Comm. 1981, 748-749.
[2] R. H. J. Smith, C. J. Michejda, Synthesis 1983, 476-477.
[3] R. H. J. Smith, B. D. Wladowski, A. F. Mehl, M. J. Cleveland, E. A. Rudrow, G. N. Chmurny, C. J. Michejda, J. Org. Chem. 1989, 54, 1036-1042.
[4] D. H. Sieh, D. J. Wilbur, C. J. Michejda, J. Am. Chem. Soc. 1980, 3883-3887.
[5] Soussi, K., Mishra, S, Jeanneau, E, Millet, J.-M., Daniele, S., Dalton Transactions 2017, 46, 38, 13055 bis 13064
[6] C. Lego, B. Neumüller, Z. Anorg. Allg. Chem. 2011, 637, 1784-1789.
[7] P. Gantzel, P. J. Walsh, Inorg. Chem. 1998, 37, 3450-3451.
[8] D. Kalden, S. Krieck, H. Görls, M. Westerhausen, Dalt. Trans. 2015, 44, 8089-8099.
[9] H. S. Lee, S.-O. Hauber, D. Vindus, M. Niemeyer, Inorg. Chem. 2008, 47, 4401-4412.
[10] S. L. Hyui, M. Niemeyer, Inorg. Chem. 2006, 45, 6126-6128.
[11] F. E. Brinckman, H. S. Haiss, R. A. Robb, Inorg. Chem. 1965, 4, 936-942.
[12] J. T. Leman, A. R. Barron, J. W. Ziller, R. M. Kren, Polyhedron 1989, 8, 1909-1912.
[13] J. T. Leman, H. A. Roman, A. R. Barron, J. Chem. Soc. Dalt. Trans. 1992, 1, 2183.
[14] M. L. Cole, A. J. Davies, C. Jones, P. C. Junk, A. I. McKay, A. Stasch, Z. Anorg. Allg. Chem. 2015, 641, 2233-2244.
[15] S. G. Alexander, M. L. Cole, C. M. Forsyth, S. K. Furfari, K. Konstas, Dalt. Trans. 2009, 2326-2336.
[16] J. T. Leman, J. Braddock-Wilking, A. J. Coolong, A. R. Barron, Inorg. Chem. 1993, 32, 4324-4336.
[17] I. A. Guzei, L. M. Liable-Sands, A. L. Rheingold, C. H. Winter, Polyhedron 1997, 16,4017-4022.
[18] V. C. Gibson, D. F. Reardon, A. K. Tomov, 2004, WO 2004063233*.*
[19] E. Hartmann, J. Strähle, J. Inorg. Gen. Chem. 1990, 583, 31-40.
[20] I. D. Brown, J. D. Dunitz, Acta Cryst. 1961, 14, 480-485.
[21] L. R. Falvello, E. P. Urriolabettia, U. Mukhopadhyay, D. Ray, Acta Cryst. Comm. 1999, C55, 170-172.
[22] H. S. Lee, M. Niemeyer, Inorg. Chim. Acta 2011, 374, 163-170.
[23] A. L. Johnson, A. M. Willcocks, S. P. Richards, Inorg. Chem. 2009, 48, 8613-8622.
[24] E. Pfeiffer, M. W. Kokkes, K. Vrieze, Transit. Met. Chem. 1979, 4, 389-393.
[25] M. Venter, I. Haiduc, L. David, O. Cozar, J. Mol. Struct. 1997, 408-409, 483-486.
[26] F. A. Cotton, X. Feng, Inorg. Chem. 1989, 28, 1180-1183.
[27] J. G. M. van der Linden, A. H. Dix, E. Pfeiffer, Inorg. Chim. Acta 1980, 39, 271-274.
[28] S. F. Colson, S. D. Robinson, Inorg. Chim. Acta Lett. 1988, 149, 13-14.
[29] S. F. Colson, S. D. Robinson, M. Motevalli, M. B. Hursthouse, Polyhedron 1988, 7, 1919-1924.
[30] J. A. Cabeza, J. M. Fernhdez-Colinas, Coord. Chem. Rev. 1993, 126, 319-336.
[31] C. J. Weiss, T. J. Marks, Dalt. Trans. 2010, 39, 6576.
[32] B. Liu, D. Cui, Dalt. Trans. 2009, 550-556.
[33] P. J. Eulgem, A. Klein, N. Maggiarosa, D. Naumann, R. W. H. Pohl, Chem. Eur. J. 2008, 14, 3727-3736.
[34] S. G. Alvarez, M. T. Alvarez, Synthesis 1997, 4, 413-414.
[35] P. Margaretha, S. Solar, O. E. Polansky, Angew. Chemie - Int. Ed. 1971, 10, 412-413.
[36] P. Margaretha, S. Solar, O. E. Polansky, Angew. Chemie 1971, 11, 410.
[37] J. C. Bottaro, P. E. Penwell, R. J. Schmitt, Synth. Commun. 1997, 27, 1465-1467.

## Patentansprüche

1. Verwendung eines Metallkomplexes, der mindestens einen Liganden **L** der Formel R¹-N₃-R² aufweist, wobei R¹ und R² Kohlenwasserstoffreste sind, zum Abscheiden des Metalls oder einer Verbindung des Metalls aus der Gasphase.

2. Verwendung gemäß Anspruch 1, wobei der Metallkomplex mindestens einen Liganden **L** der Formel R¹-N₃-R² und mindestens einen weiteren Liganden **X** aufweist, der ausgewählt ist aus H, Halogen, CO und Kohlenwasserstoffliganden.

3. Verwendung gemäß mindestens einem der vorhergehenden Ansprüche, wobei der Metallkomplex mindestens einen Liganden **L** der Formel R¹-N₃-R² aufweist, bei dem R¹ und R² Alkylreste sind.

4. Verwendung gemäß mindestens einem der vorhergehenden Ansprüche, wobei der Metallkomplex die Formel (1) aufweist:
Mₓ[(L¹)ₐ(L²)_{b}(L³)_{c}X_{d}] (1)
wobei
die Liganden **L,** nämlich L¹, L² und L³, unabhängig voneinander ausgewählt sind aus Resten der Formel R¹-N₃-R², wobei R¹ und R² Kohlenwasserstoffreste sind,
wobei mindestens bei L¹ die Reste R¹ und R² Alkylreste sind,
**X** unabhängig voneinander ausgewählt ist aus H, Halogen, CO und Kohlenwasserstoffliganden,
x eine ganze Zahl zwischen 1 und 4 ist,
a, b, c und d ganze Zahlen sind, wobei
die Summe a + b + c + d mindestens x ist und nicht größer als 12 ist,
a mindestens 1 ist, und
b, c und d = 0 sein können.

5. Verwendung gemäß mindestens einem der vorhergehenden Ansprüche, wobei der Metallkomplex homoleptisch ist und ausschließlich Liganden **L** der Formel R¹-N₃-R² aufweist.

6. Verwendung gemäß mindestens einem der vorhergehenden Ansprüche, wobei der Metallkomplex heteroleptisch ist.

7. Verwendung gemäß mindestens einem der vorhergehenden Ansprüche, wobei die Reste R¹ und R² unabhängig voneinander 1 bis 30 C-Atome aufweisen.

8. Verwendung gemäß mindestens einem der vorhergehenden Ansprüche, wobei alle Reste R¹ und R² des Metallkomplexes Alkylreste sind.

9. Verwendung gemäß Anspruch 8, wobei die Reste R¹ und R² unabhängig voneinander 1 bis 15 C-Atome aufweisen.

10. Verwendung gemäß Anspruch 8 oder 9, wobei alle oder ein Teil der Reste R¹ und/oder R² verzweigte und/oder zyklische Alkylreste sind.

11. Verwendung gemäß Anspruch 10, wobei alle oder ein Teil der Reste R¹ und/oder R² tert-Butyl sind.

12. Verwendung mindestens einem der vorhergehenden Ansprüche, wobei der Metallkomplex mindestens einen Liganden **L** aufweist, der *tert*-Butyl-N₃-*tert*-Butyl oder *tert*-Butyl-N₃-Methyl ist.

13. Verwendung gemäß Anspruch 12, wobei alle Liganden **L** des Metallkomplexes *tert-*Butyl-N₃-*tert*-Butyl oder *tert*-Butyl-N₃-Methyl sind.

14. Verwendung mindestens einem der vorhergehenden Ansprüche 2 bis 13, wobei mindestens ein **X** ein Kohlenwasserstoffligand ist, der ausgewählt ist aus Alkyl oder Alkenyl mit 1 bis 12 Kohlenstoffatomen und aromatischen Kohlenwasserstoffen mit 5 bis 30 C-Atomen.

15. Verwendung gemäß mindestens einem der vorhergehenden Ansprüche, wobei das Metall M ausgewählt ist aus In, Co, Cu, Ru, Al, Ga, Tl, und La.

16. Verwendung gemäß Anspruch 15, wobei das Metall M ausgewählt ist aus In, Co, Cu, La und Ru.

17. Verwendung gemäß mindestens einem der vorhergehenden Ansprüche, wobei der Metallkomplex die Formel (2) aufweist:
M[(L¹)ₐX_{d}] (2)
wobei
L¹ die Formel R¹-N₃-R² aufweist, wobei R¹ und R² Alkylreste mit 1 bis 12 C-Atomen sind,
X ausgewählt ist aus H, Halogen, CO und Alkyl mit 1 bis 12 C-Atomen,
a = 2 oder 3 ist,
d = 0 oder 1 ist, und
M ausgewählt ist aus In, Co, Cu, Al, Ga, Tl und La.

18. Verwendung gemäß Anspruch 17, wobei der Metallkomplex eine der Formeln (3) bis (5) aufweist:
M[(L¹)₂X] (3)
wobei
L¹ die Formel R¹-N₃-R² aufweist, wobei R¹ und R² Alkylreste mit 1 bis 12 C-Atomen sind,
X ausgewählt ist aus H und Alkyl mit 1 bis 12 C-Atomen, und
M = Al oder Ga ist;
M[(L¹)₃] (4)
wobei
L¹ die Formel R¹-N₃-R² aufweist, wobei R¹ und R² Alkylreste mit 1 bis 12 C-Atomen sind, und
M = In, Tl oder La ist;
Mₓ(L¹)ₐ (5)
wobei
L¹ die Formel R¹-N₃-R² aufweist, wobei R¹ und R² Alkylreste mit 1 bis 12 C-Atomen sind,
x eine ganze Zahl zwischen 1 und 4 ist, und
a eine ganze Zahl zwischen 2 und 8 ist, mit der Maßgabe, dass a/x = 1 oder 2 ist, und
M = Co oder Cu ist.

19. Verwendung gemäß mindestens einem der Ansprüche 1 bis 16, wobei der Metallkomplex die Formel (6) aufweist:
(Ru[(L¹)X¹X²] (6)
wobei
L¹ die Formel R¹-N₃-R² aufweist, wobei R¹ und R² Alkylreste mit 1 bis 12 C-Atomen sind,
X¹ ein aromatischer Kohlenwasserstoffligand mit 5 bis 30 C-Atomen ist, und
X² ausgewählt ist aus keinem Rest, H, Halogen, CO und Alkyl mit 1 bis 6 C-Atomen.

20. Verwendung gemäß mindestens einem der vorhergehenden Ansprüche, wobei der Metallkomplex sublimierbar oder verdampfbar ist, ohne dass eine Zersetzung erfolgt.

21. Verwendung gemäß mindestens einem der vorhergehenden Ansprüche, wobei das Molekulargewicht des Metallkomplexes kleiner ist als kleiner als 600 g/Mol.

22. Verwendung gemäß mindestens einem der vorhergehenden Ansprüche, wobei der Metallkomplex bei 100°C thermisch stabil ist und/oder in einem Temperaturbereich von 100°C bis 400°C zerfällt, wobei die Temperatur bei Normaldruck oder bei reduziertem Druck im Bereich von 10⁻³ bis 900 mbar ermittelt wird.

23. Verwendung gemäß mindestens einem der vorhergehenden Ansprüche, wobei das Abscheiden des Metalls oder der Verbindung des Metalls aus der Gasphase bei reduziertem Druck im Bereich von 10⁻³ bis 900 mbar, bevorzugt im Bereich von 10⁻² bis 1 mbar, und insbesondere 10⁻² mbar erfolgt.

24. Verfahren zur Herstellung von beschichteten Substraten, umfassend die Schritte:
(a) Bereitstellen eines Metallkomplexes gemäß mindestens einem der vorhergehenden Ansprüche, und
(b) Abscheiden des Metalls oder einer Verbindung des Metalls auf der Oberfläche des Substrats durch metallorganische Gasphasenabscheidung.

25. Verfahren oder Verwendung gemäß mindestens einem der vorhergehenden Ansprüche, wobei das Verfahren eine metallorganische chemische Gasphasenabscheidung (MOCVD) ist.

26. Verfahren oder Verwendung gemäß mindestens einem der vorhergehenden Ansprüche, wobei das Verfahren eine metallorganische Gasphasenepitaxie (MOVPE) ist.

27. Verfahren oder Verwendung gemäß mindestens einem der vorhergehenden Ansprüche, wobei die Verbindung des Metalls aus Halbleiterverbindungen, Legierungen, Nitriden, Phosphiden, Arseniden und Siliziden ausgewählt ist.

28. Verfahren oder Verwendung gemäß mindestens einem der vorhergehenden Ansprüche, wobei der Metallkomplex sublimiert oder verdampft wird, ohne dass eine Zersetzung erfolgt.

29. Verfahren oder Verwendung gemäß mindestens einem der vorhergehenden Ansprüche, wobei sich der Metallkomplex in der Gasphase zersetzt bei einer Temperatur, die nicht mehr als 100°C oberhalb der Sublimationstemperatur oder Verdampfungstemperatur liegt.

30. Verfahren zur Herstellung eines Metallkomplexes, wobei der Metallkomplex ein solcher gemäß mindestens einem der Ansprüche 1 bis 23 ist, umfassend die Schritte
(A) Bereitstellen einer Verbindung der Formel R¹-(N₃)A-R², wobei A ausgewählt ist aus H oder einem Alkalimetall, insbesondere Li, Na oder K, und
(B) in Kontakt bringen mit einer Verbindung des Metalls.

31. Verfahren gemäß Anspruch 30, wobei die Verbindung des Metalls in Schritt (B) ausgewählt ist aus einem Metallsalz, einer metallorganischen Verbindung oder einem Metallkomplex des Metalls, der nicht einen Liganden L aufweist.

32. Verfahren zur Herstellung einer Verbindung der Formel R¹-(N₃)A-R² in einem Reaktionsgemisch, das die Verbindungen R¹-N₃ und AR² enthält, wobei A ein Alkalimetall ist.

33. Verfahren gemäß Anspruch 30 oder 31, wobei in einem vorhergehenden Schritt die Verbindung der Formel R¹-(N₃)A-R², wobei A ein Alkalimetall ist, in einem Verfahren gemäß Anspruch 32 hergestellt wird.

34. Verfahren gemäß mindestens einem der Ansprüche 30 bis 33, wobei das A = Li ist.

35. Metallkomplex der Formel (1):
Mₓ[(L¹)ₐ(L²)_{b}(L³)_{c}X_{d}] (1)
wobei
die Liganden **L,** nämlich L¹, L² und L³, unabhängig voneinander ausgewählt sind aus Resten der Formel R¹-N₃-R², wobei R¹ und R² Kohlenwasserstoffreste sind,
wobei mindestens bei L¹ die Reste R¹ und R² Alkylreste sind,
der Ligand **X** ausgewählt ist aus Halogen, H, CO und Kohlenwasserstoffliganden,
x eine ganze Zahl zwischen 1 und 4 ist,
a, b, c und d ganze Zahlen sind, wobei
die Summe a + b + c + d mindestens x ist und nicht größer als 12 ist,
a mindestens 1 ist, und
b, c und d = 0 sein können,
wobei mindestens eine der folgenden Bedingungen (i) bis (ii) erfüllt ist:
(i) M ist ausgewählt aus Metallen der VIII. Nebengruppe und den Lanthaniden des Periodensystems der Elemente,
(ii) mindestens ein Ligand **L** weist mindestens einen Rest R¹ oder R² auf, der *tert-*Butyl ist.

36. Metallkomplex gemäß Anspruch 35, der eine der Formeln (7) bis (20) aufweist:

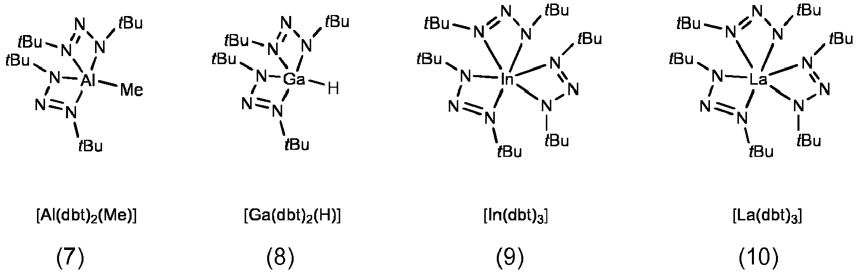

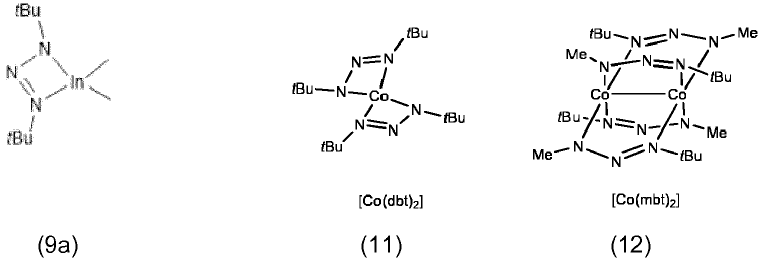

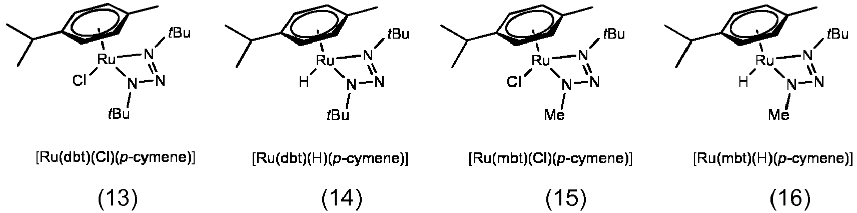

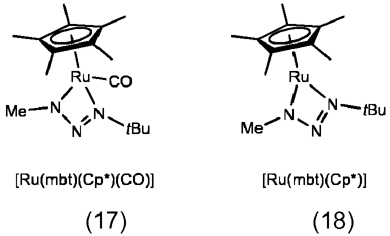

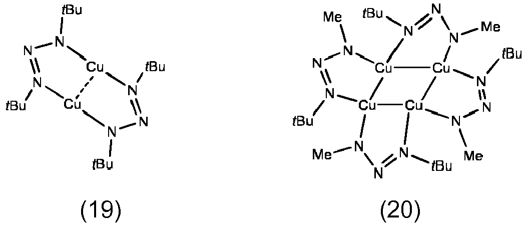

37. Verbindung, ausgewählt aus *tert*-Butyl-(N₃)H-CH₃ und Organoalkalimetallsalzen der Formel R¹-(N₃)A-R², wobei R¹ und R² Alkylreste sind und A ein Alkalimetall ist, insbesondere Li, Na oder K.

38. Organoalkalimetallsalz gemäß Anspruch 37, die eine der Formeln (21) bis (23) aufweist:
